(19) Europäisches Patentamt
European Patent Office
Office européen des brevets

(11) **EP 4 122 382 B1**

(12) **EUROPEAN PATENT SPECIFICATION**

(45) Date of publication and mention
of the grant of the patent:
**21.01.2026 Bulletin 2026/04**

(21) Application number: **20925995.1**

(22) Date of filing: **18.11.2020**

(51) International Patent Classification (IPC):
*A61B 5/021* (2006.01)   *A61B 5/026* (2006.01)
*A61B 5/00* (2006.01)   *G16H 50/20* (2018.01)

(52) Cooperative Patent Classification (CPC):
**A61B 5/725; A61B 5/02108; A61B 5/0261;**
**A61B 5/7257; A61B 5/726; A61B 5/7267;**
**G16H 50/20;** A61B 5/7275

(86) International application number:
**PCT/CN2020/129646**

(87) International publication number:
**WO 2021/184805 (23.09.2021 Gazette 2021/38)**

(54) **BLOOD PRESSURE PREDICTION METHOD AND DEVICE USING MULTIPLE DATA SOURCES**

BLUTDRUCKVORHERSAGEVERFAHREN UND -VORRICHTUNG MIT MEHREREN
DATENQUELLEN

PROCÉDÉ ET DISPOSITIF DE PRÉDICTION DE PRESSION SANGUINE UTILISANT UNE
PLURALITÉ DE SOURCES DE DONNÉES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.03.2020 CN 202010189221**

(43) Date of publication of application:
**25.01.2023 Bulletin 2023/04**

(73) Proprietor: **Lepu Medical Technology (Beijing)**
**Co., Ltd.**
**Beijing 102200 (CN)**

(72) Inventors:
• **SUN, Hongdai**
**Beijing 102200 (CN)**
• **CAO, Jun**
**Beijing 102200 (CN)**

(74) Representative: **Glück Kritzenberger**
**Patentanwälte PartGmbB**
**Franz-Mayer-Str. 16a**
**93053 Regensburg (DE)**

(56) References cited:
CN-A- 106 073 729     CN-A- 106 821 355
CN-A- 108 498 089     CN-A- 109 833 034
CN-A- 111 358 455     US-A1- 2019 104 951
US-A1- 2020 015 755

• BAEK SANGHYUN ET AL: "End-to-End Blood
Pressure Prediction via Fully Convolutional
Networks", IEEE ACCESS, vol. 7, 31 December
2019 (2019-12-31), pages 185458 - 185468,
XP011763871, DOI: 10.1109/
ACCESS.2019.2960844
• SHIMAZAKI SHOTA ET AL: "Cuffless Blood
Pressure Estimation from only the Waveform of
Photoplethysmography using CNN", 2019 41ST
ANNUAL INTERNATIONAL CONFERENCE OF
THE IEEE ENGINEERING IN MEDICINE AND
BIOLOGY SOCIETY (EMBC), IEEE, 23 July 2019
(2019-07-23), pages 5042 - 5045, XP033624618,
DOI: 10.1109/EMBC.2019.8856706

## Description

The application claims priority to Chinese Patent Application No. 202010189221.3, entitled "BLOOD PRESSURE PREDICTION METHOD AND DEVICE USING MULTIPLE DATA SOURCES", filed with the China National Intellectual Property Administration on March 17, 2020.

## BACKGROUND OF THE INVENTION

### 1. Technical Field

The invention relates to the technical field of electrophysiological signal processing, in particular to a blood pressure prediction method and device using multiple data sources.

### 2. Description of Related Art

Photoplethysmograph (PPG) signals are a set of signals used for recording the change of light intensity by identifying the light intensity of a specific light source with a light sensor. When the heart beats, the blood flow of unit area in blood vessels changes periodically, the blood volume changes correspondingly, and a PPG signal reflecting the light absorption capacity of blood also changes periodically. One cardiac cycle comprises two time periods: a systole period and a diastole period. In the systole period, the heart acts on blood in the whole body to make the pressure and blood flow volume in the blood vessels change continuously and periodically, and at this moment, blood in the blood vessels absorbs the most light. In the diastole period, the pressure applied to the blood vessels is relatively low, and at this moment, blood pushed to the whole body in the previous systole period cyclically impacts the heart valves to reflect and refract light to some extent, so less light energy is absorbed by blood in the blood vessels in the diastole period. Thus, the blood pressure can be predicted by analyzing the PPG signal waveform capable of reflecting the light energy absorbed by blood in the blood vessels.

In actual application, we find that the PPG signal used for blood pressure prediction may be acquired in different ways. Specifically, the PPG signal may be acquired directly through a PPG signal acquisition device or be acquired indirectly by recording a video of the skin surface of a test subject. If the PPG signal is acquired directly, the PPG signal may be distorted under the influence of factors such as the sensitivity of a sensor, the physiological status of the test subject, and signal interference in the environment. If the PPG signal is extracted from a video by normalized transform of red and green light channel data, the PPG signal may also be distorted due to factors such as the light intensity of a photographing environment. A blood pressure prediction result obtained by using the distorted PPG signal will drastically deviate from the actual blood pressure and may be even incorrect. In general, blood-pressure prediction from PPG measurements and modelling is known for example from BAEK SANGHYUN ET AL: "End-to-End Blood Pressure Prediction via Fully Convolutional Networks",IEEE ACCESS, vol. 7, 31 December 2019.

## BRIEF SUMMARY OF THE INVENTION

The objective of the invention is to overcome the defects of the prior art by providing a blood pressure prediction method and device using multiple data sources. According to the blood pressure prediction method and device, two signal filtering and shaping methods are provided for directly acquired PPG signals, and a video quality detection and normalized signal conversion method is provided for indirectly generated PPG signal, and a uniform standard PPG data sequence is finally generated for blood pressure prediction; and the embodiments of the invention provide two optional convolutional neural networks (CNN) models for blood pressure prediction. By adoption of the method and device provided by the embodiments of the invention, the capacity to process various PPG signal data sources and the capacity to manage various blood pressure prediction models of an application are improved, and the compatibility with various data sources for blood pressure prediction of the application is improved.

To fulfill the above objective, in a first aspect, the embodiments of the invention provide a blood pressure prediction method using multiple data sources, comprising:

Acquiring a data source identifier and original data from an upper computer, wherein the data source identifier is one of a first-class PPG original signal identifier, a second-class PPG original signal identifier and a third-class PPG video identifier; and the original data is one of a first-class PPG original data, a second-class PPG original signal and third-class PPG video data, and corresponds to the data source identifier;

Preprocessing the original data according to the data source identifier; when the data source identifier is the first-class PPG original signal identifier, performing normalized filtering on the first-class PPG original signal to generate a standard PPG data sequence; when the data source identifier is the second-class PPG original signal identifier, performing baseline drift removal and normalized filtering on the second-class PPG original signal to generate the standard PPG data sequence; and when the data source identifier is the third-class PPG video identifier, performing video quality detection and normalized signal conversion on the third-class PPG video data to generate the standard PPG data sequence;

Acquiring a CNN model identifier, wherein the CNN model identifier is a first-class CNN identifier or a second-class CNN identifier; and

Selecting a corresponding CNN model to perform blood pressure prediction on the standard PPG data sequence according to the CNN model identifier; when the CNN model identifier is the first-class CNN identifier, selecting a first-class CNN model to perform blood pressure prediction on the standard PPG data sequence; or when the CNN model identifier is the second-class CNN identifier, selecting a second-class CNN model to perform wavelet transform-based blood pressure prediction on the standard PPG data sequence.

When the data source identifier is the first-class PPG original signal identifier, performing normalized filtering on the first-class PPG original signal to generate a standard PPG data sequence, specifically comprises:

When the data source identifier is the first-class PPG original signal identifier, performing data sampling on the first-class PPG original signal according to a preset first-class signal sampling threshold to generate a first-class PPG sampling data sequence $(X_1, X_2...X_i...X_M)$, wherein the first-class PPG sampling data sequence $(X_1, X_2...X_i...X_m)$ comprises M first-class PPG sampling data $X_i$, M is an integer, and i ranges from 1 to M;

Performing normalized filtering on the first-class PPG sampling data sequence $(X_1, X_2...X_i...X_M)$ to generate a first process sequence $(Y_1, Y_2...Y_i...Y_m)$; when i is 1, setting $Y_i = X_i$; or, when i is greater than 1, setting $Y_i$ according to a formula $Y_i = \frac{a \times Y_{i-1}}{b} + \frac{X_i}{b \times c}$ wherein the first process sequence $(Y_1, Y_2...Y_i...Y_M)$ comprises M first process data $Y_i$, a and b are preset first-class filtering constants, and c is a gain coefficient of the first-class PPG original signal; and

Setting the standard PPG data sequence as the first process sequence $(Y_1, Y_2...Y_i...Y_M)$.

Preferably, when the data source identifier is the second-class PPG original signal identifier, performing baseline drift removal and normalized filtering on the second-class PPG original signal to generate the standard PPG data sequence, comprises:

When the data source identifier is the second-class PPG original signal identifier, performing data sampling on the second-class PPG original signal according to a preset second-class signal sampling threshold to generate a second-class PPG sampling data sequence $(S_1, S_2...S_j...S_N)$, wherein the second-class PPG sampling data sequence $(S_1, S_2...S_j...S_N)$ comprises N second-class PPG sampling data $S_j$, N is an integer, and j ranges from 1 to N;

Performing baseline drift removal and filtering on the second-class PPG sampling data sequence $(S_1, S_2...S_j...S_N)$ to generate a second process sequence $(T_1, T_2...T_j...T_N)$; when j is 1, setting $T_i = S_i$; or, when j is greater than 1, setting $T_j$ according to a formula $T_i = e_1 \times S_i + e_2 \times S_{j-1} - e_3 \times T_{i-1}$, wherein the second process sequence $(T_1, T_2...T_j...T_n)$ comprises N second process data $T_j$, and $e_1$, $e_2$, and $e_3$ are all preset high-pass filtering coefficients;

Extracting a maximum value from the second process sequence $(T_1, T_2...T_j...T_N)$ to generate a maximum reference value max, and extracting a minimum value from the second process sequence $(T_1, T_2...T_j...T_N)$ to generate a minimum reference value min;

Performing normalized filtering on the second process sequence $(T_1, T_2...T_j...T_n)$ to generate a third process sequence $(P_1, P_2...P_j...P_N)$ specifically by setting $P_j$ according to a formula $P_j = \frac{T_j - min}{max - min}$ , wherein the third process sequence $(P_1, P_2...P_j...P_N)$ comprises N third process data $P_j$; and

Setting the standard PPG data sequence as the third process sequence $(P_1, P_2...P_j...P_N)$.

When the data source identifier is the third-class PPG video identifier, performing video quality detection and normalized signal conversion on the third-class PPG video data to generate the standard PPG data sequence, specifically comprises:

When the data source identifier is the third-class PPG video identifier, performing video data frame image extraction on the third-class PPG video data to generate a third-class PPG video frame image sequence, wherein the third-class PPG video frame image sequence comprises multiple third-class PPG video frame images;

Performing one-dimensional red light signal extraction on all the third-class PPG video frame images in the third-class PPG video frame image sequence according to a preset red light pixel threshold range to generate a first red light digital signal, and performing one-dimensional green light signal extraction on all the third-class PPG video frame images in the third-class PPG video frame image sequence according to a preset green light pixel threshold range to generate a first green light digital signal;

According to a preset band-pass filtering frequency threshold range, performing band-pass filtering preprocessing on the first red light digital signal to generate a second red light digital signal, and performing band-pass filtering preprocessing on the first green light digital signal to generate a second green light digital signal;

Performing maximum frequency difference determination on the second red light digital signal and the second green light digital signal to generate a first determination result;

When the first determination result is an up-to-standard signal identifier, performing signal-to-noise ratio determination on the second red light digital signal and the second green light digital signal to generate a second determination result; and

When the second determination result is the up-to-standard signal identifier, performing normalized PPG signal data sequence generation on the second red light digital signal and the second green light digital signal to generate the standard PPG data sequence.

Further, performing one-dimensional red light signal extraction on all the third-class PPG video frame images in the third-class PPG video frame image sequence according to a preset red light pixel threshold range to generate a first red light digital signal, and performing one-dimensional green light signal extraction on all the third-class PPG video frame images in the third-class PPG video frame image sequence according to a preset green light pixel threshold range to generate a first green light digital signal, specifically comprise:

Step 51, initializing the first red light digital signal to be null, initializing the first green light digital signal to be null, initializing a first index to 1, and initializing a first total number to a total number of the third-class PPG video frame images in the third-class PPG video frame image sequence;

Step 52, setting a first-index frame image as the third-class PPG video frame image, corresponding to the first index, in the third-class PPG video frame image sequence;

Step 53, collecting all pixels, meeting the red light pixel threshold range, in the first-index frame image to generate a red pixel set, calculating a total number of the pixels in the red pixel set to generate a red pixel total number, calculating the sum of pixel values of all the pixels in the red pixel set to generate a red pixel value sum, and generating first-index frame red light channel data according to a quotient obtained by dividing the red pixel value sum by the red pixel total number; and adding signal points into the first red light digital signal using the first-index frame red light channel data as signal point data;

Step 54, collecting all pixels, meeting the green light pixel threshold range, in the first-index frame image to generate a green pixel set, calculating a total number of the pixels in the green pixel set to generate a green pixel total number, calculating the sum of pixel values of all the pixels in the green pixel set to generate a green pixel value sum, and generating first-index frame green light channel data according to a quotient obtained by dividing the green pixel value sum by the green pixel total number; and adding signal points into the first green light digital signal using the first-index frame green light channel data as signal point data;

Step 55, increasing the first index by 1;

Step 56, determining whether the first index is greater than the first total number; if the first index is less than or equal to the first total number, performing Step 52; or, if the first index is greater than the first total number, performing Step 57; and

Step 57, transferring the first red light digital signal to an upper processing process as a one-dimensional red light signal extraction result, and transferring the first green light digital signal to an upper processing process as a one-dimensional green light signal extraction result.

Further, performing maximum frequency difference determination on the second red light digital signal and the second green light digital signal to generate a first determination result, specifically comprises:

Performing digital signal time domain-frequency domain conversion on the second red light digital signal through discrete Fourier transform to generate a red light frequency domain signal, and performing digital signal time domain-frequency domain conversion on the second green light digital signal through discrete Fourier transform to generate a green light frequency domain signal;

Extracting a maximum-energy frequency from the red light frequency domain signal to generate a maximum red light frequency, and extracting a maximum-energy frequency from the green light frequency domain signal to generate a maximum green light frequency;

Calculating a frequency difference between the maximum red light frequency and the maximum green light frequency to generate a maximum red-green frequency difference; and

When the maximum red-green frequency difference does not exceed a preset maximum frequency difference threshold range, setting the first determination result as the up-to-standard signal identifier.

Further, when the first determination result is an up-to-standard signal identifier, performing signal-to-noise ratio determination on the second red light digital signal and the second green light digital signal to generate a second determination result, specifically comprises:

When the first determination result is the up-to-standard signal identifier, according to a preset band-stop filtering frequency threshold range, removing valid signal points, meeting the band-stop filtering frequency threshold range, from the second red light digital signal through multi-order Butterworth band-stop filtering to generate a red light noise signal, and removing valid signal points, meeting the band-stop filtering frequency threshold range, from the second green light digital signal through multi-order Butterworth band-stop filtering to generate a green light noise signal;

Calculating signal energy of the second red light digital signal to generate red light signal energy, calculating signal energy of the red light noise signal to generate red light noise energy, generating valid red light signal energy according to a difference between the red light signal energy and the red light noise energy, and generating a red light signal-to-noise ratio according to a ratio of the valid red light signal energy to the red light noise energy;

Calculating signal energy of the second green light digital signal to generate green light signal energy, calculating signal energy of the green light noise signal to generate green light noise energy, generating valid green light signal energy according to a difference between the green light signal energy and the green light noise energy, and generating a green light signal-to-noise ratio according to a ratio of the valid green light signal energy to the green light noise energy; and

When any one of the red light signal-to-noise ratio and the green light signal-to-noise ratio is greater than or equal to the signal-to-noise threshold, setting the second determination result as the up-to-standard signal identifier.

Further, when the second determination result is the up-to-standard signal identifier, performing normalized PPG signal data sequence generation on the second red light digital signal and the second green light digital signal to generate the standard PPG data sequence, specifically comprises:
When the second determination result is the up-to-standard signal identifier, performing signal data normalization processing on the second red light digital signal and the second green light digital signal respectively to generate a normalized red light signal and a normalized green light signal; setting a red light data sequence of the standard PPG data sequence as the normalized red light signal, and setting a green data sequence of the standard PPG data sequence as the normalized green light signal, wherein the standard PPG data sequence comprises the red light data sequence and the green light data sequence.

Preferably,

The first-class CNN model comprises multiple CNN network layers and a fully connected layer, and each CNN network layer comprises a convolutional layer and a pooling layer;
The second-class CNN model comprises a two-dimensional convolutional layer, a maximum pooling layer, a batch normalization layer, an activation layer, an add layer, a global average pooling layer, a dropout layer and a fully connected layer.

Preferably, when the CNN model identifier is the first-class CNN identifier, selecting a first-class CNN model to perform blood pressure prediction on the standard PPG data sequence, specifically comprises:

When the CNN model identifier is the first-class CNN identifier, performing first-class CNN model input data conversion on the standard PPG data sequence according to a preset first-class CNN input width threshold to generate an input data four-dimensional tensor;
According to a preset convolutional layer number threshold, performing multilayer convolution and pooling calculation on the input data four-dimensional tensor by means of the CNN network layers of the first-class CNN model to generate a feature data four-dimensional sensor;
Performing two-dimensional matrix construction of fully connected layer input data two-dimensional matrix construction of fully connected layer input data according to the feature data four-dimensional tensor to generate an input data two-dimensional matrix, and performing feature data regression calculation on the input data two-dimensional matrix by means of the fully connected layer of the first-class CNN model to generate a blood pressure regression data two-dimensional matrix;
Acquiring a preset prediction mode identifier, wherein the prediction mode identifier is a mean prediction identifier or a dynamic prediction identifier;
When the prediction mode identifier is the mean prediction identifier, performing mean blood pressure calculation on the two-dimensional matrix of blood pressure regression data to generate a mean blood pressure prediction data pair, wherein the mean blood pressure prediction data pair comprises mean systolic pressure prediction data and mean diastolic pressure prediction data; or

When the prediction mode identifier is the dynamic prediction identifier, performing dynamic blood pressure data extraction on the two-dimensional matrix of blood pressure regression data to generate a one-dimensional data sequence of dynamic blood pressure prediction .

Preferably, when the CNN model identifier is the second-class CNN identifier, selecting a second-class CNN model to perform wavelet transform-based blood pressure prediction on the standard PPG data sequence, specifically comprises:

When the data source identifier is the first-class PPG original signal identifier or the second-class PPG original signal identifier and the CNN model identifier is the second-class CNN identifier, performing data fragment division on the standard PPG data sequence to generate standard PPG data fragments;

Acquiring a preset wavelet basis type, a scalability factor array and a mobile factor array, wherein the scalability factor array comprises H scalability factors, the mobile factor array comprises L mobile factors, and H and L are both integers;

Performing signal decomposition on the standard PPG data fragments through continuous wavelet transform according to the scalability factors in the scalability factor array, the mobile factors in the scalability factor array and the wavelet basis type to generate a PPG wavelet coefficient matrix [H, L];

Transforming the PPG wavelet coefficient matrix into a real matrix through a modulo operation on matrix elements, and performing normalization processing on values of matrix elements in the real matrix to generate a PPG normalized matrix [H, L];

Acquiring an RGB color palette matrix, and performing PPG time-frequency tensor conversion on the PPG normalized matrix [H, L] according to the RGB color palette matrix to generate a PPG time-frequency three-dimensional tensor [H, L, 3];

According to a preset second-class CNN input width threshold, performing tensor shape reconstruction on the PPG time-frequency three-dimensional tensor [H, L, 3] through a bicubic interpolation algorithm to generate a PPG convolutional three-dimensional tensor [K, K, 3], wherein K is the second-class CNN input width threshold; and

Performing blood pressure prediction on the PPG convolutional three-dimensional tensor [K, K, 3] using the second-class CNN model to generate a PPG blood pressure prediction data pair, wherein the PPG blood pressure prediction data pair comprises PPG systolic pressure prediction data and PPG diastolic pressure prediction data.

According to the blood pressure prediction method using multiple data sources provided by the embodiments of the invention in the first aspect, two signal filtering and shaping methods are provided for directly acquired PPG signals, and a video quality detection and normalized signal conversion method is provided for indirectly generated PPG signal, and a uniform standard PPG data sequence is generated for blood pressure prediction; and during blood pressure prediction, different blood pressure prediction modes are provided for blood pressure prediction according to a CNN model identifier. By adoption of the method and device provided by the embodiments of the invention, the PPG signal preprocessing capacity and compatibility of an application are improved, and different blood prediction methods are provided.

In a second aspect, the embodiments of the invention provide equipment, comprising a memory and a processor, wherein the memory is used to store a program, and the processor is used to implement the method in the first aspect and in all implementations of the first aspect.

In a third aspect, the embodiments of the invention provide a computer program product comprising instructions, wherein the computer program product enables a computer to implement the method in the first aspect and in all implementations of the first aspect when running on the computer.

In a fourth aspect, the embodiments of the invention provide a computer-readable storage medium having a computer program stored therein, wherein when the computer program is executed by a processor, the method in the first aspect and in all implementations of the first aspect is implemented.

BRIEF DESCRIPTION OF THE SEVERAL VIEWS OF THE DRAWINGS

FIG. 1 is a schematic diagram of a blood pressure prediction method using multiple data sources according to Embodiment 1 of the invention;
FIG. 2 is a schematic diagram of PPG signals before and after filtering according to one embodiment of the invention;

FIG. 3 is a schematic diagram of a method for performing video quality detection on third-class PPG video data according to Embodiment 2 of the invention;

FIG. 4 is a structural diagram of a blood pressure prediction device using multiple data sources according to Embodiment 3 of the invention.

DETAILED DESCRIPTION OF THE INVENTION

To gain a better understanding of the purposes, technical solutions and advantages of the invention, the invention will be described in further detail below in conjunction with the accompanying drawings. Clearly, the embodiments in the following description are merely illustrative ones, and are not all possible ones of the invention. The scope of the invention is defined by the claims.

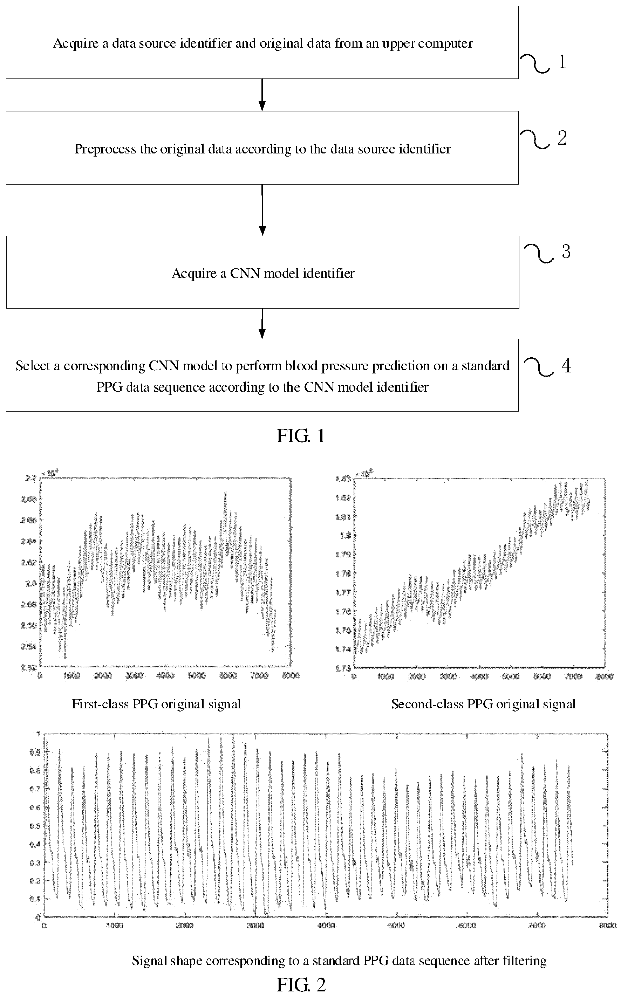

As shown in FIG. 1 , it is a schematic diagram of a blood pressure prediction method using multiple data sources provided by Embodiment 1 of the invention, the method comprises the following steps:

Step 1, a data source identifier and original data are acquired from an upper computer.

Wherein, the data source identifier is one of a first-class PPG original signal identifier, a second-class PPG original signal identifier and a third-class PPG video identifier; and the original data is one of a first-class PPG original data, a second-class PPG original signal and third-class PPG video data, and corresponds to the data source identifier;

Here, to guarantee the compatibility with various PPG data source acquisition approaches, the data source identifier is set to distinguish the type of acquired original data:

Table 1

| Data source identifier | Original data |
| --- | --- |
| First-class PPG original signal identifier | First-class PPG original data |
| Second-class PPG original signal identifier | Second-class PPG original signal |
| Third-class PPG video identifier | Third-class PPG video data |

As shown in FIG. 2 it is a schematic diagram of PPG signals before and after filtering according to this embodiment of the invention, the first-class PPG original signal and the second-class PPG original signal are generally acquired from the skin surface of a test subject through a PPG signal acquisition device, wherein signals with a non-obvious horizontal baseline drift are classified as first-class PPG original signals, and signals with an obvious horizontal baseline drift are classified as second-class PPG original signals. Generally, the third-class PPG video data is obtained by photographing the skin surface of the test subject through a video recording device and is of a common video format.

Step 2, the original data is preprocessed according to the data source identifier;

Step 2 comprises: Step 21, when the data source identifier is the first-class PPG original signal identifier, normalized filtering is performed on the first-class PPG original signal to generate a standard PPG data sequence;

Step 21 comprises: Step 211, when the data source identifier is the first-class PPG original signal identifier, data sampling is performed on the first-class PPG original signal according to a preset first-class signal sampling threshold to generate a first-class PPG sampling data sequence $(X_1, X_2...X_i...X_m)$;

Wherein, the first-class PPG sampling data sequence $(X_1, X_2...X_i...X_M)$ comprises M first-class PPG sampling data $X_i$, M is an integer, and i ranges from 1 to M;

Here, considering that the first-class PPG original signal may not be subjected to digital conversion, sampling is performed before filtering to realize standardized processing of the signal;

Step 212, normalized filtering is performed on the first-class PPG sampling data sequence $(X_1, X_2...X_i...X_M)$ to generate a first process sequence $(Y_1, Y_2...Y_i...Y_M)$;

When i is 1, $Y_i = X_i$ is set;

When i is greater than 1, $Y_i$ is set according to a formula

$$Y_i = \frac{a \times Y_{i-1}}{b} + \frac{X_i}{b \times c};$$

Wherein, the first process sequence $(Y_1, Y_2...Y_i...Y_M)$ comprises M first process data $Y_i$, a and b are preset first-class filtering constants, and c is a gain coefficient of the first-class PPG original signal;

Here, normalized filtering is performed on the first-class PPG original signal by adjusting the relative amplitude of each data point of the first-class PPG original signal, and the signal shape and amplitude after filtering are shown in FIG. 2 which is a schematic diagram of the PPG signal before and after filtering according to this embodiment of the invention;

Step 213, the standard PPG data sequence is set as the first process sequence $(Y_1, Y_2...Y_j...Y_M)$, and Step 3 is performed;

Step 22, when the data source identifier is the second-class PPG original signal identifier, baseline drift removal and normalized filtering are performed on the second-class PPG original signal to generate a standard PPG data sequence;

Step 22 comprises: Step 221, when the data source identifier is the second-class PPG original signal identifier, data sampling is performed on the second-class PPG original signal according to a preset second-class signal sampling threshold to generate a second-class PPG sampling data sequence $(S_1, S_2...S_j...S_N)$;

Wherein, the second-class PPG sampling data sequence $(S_1, S_2...S_j...S_N)$ comprises N second-class PPG sampling data $S_j$, N is an integer, and j ranges from 1 to N;

Here, considering that the second-class PPG original signal may not be subjected to digital conversion, sampling is performed before filtering to realize standardized processing of the signal;

Step 222, baseline drift removal and filtering are performed on the second-class PPG sampling data sequence $(S_1, S_2...S_j...S_N)$ to generate a second process sequence $(T_1, T_2...T_j...T_N)$;

When j is 1, $T_j = S_j$ is set;

When j is greater than 1, $T_j$ is set according to a formula

$$T_j = e_1 \times S_j + e_2 \times S_{j-1} - e_3 \times T_{j-1};$$

Wherein, the second process sequence $(T_1, T_2...T_j...T_N)$ comprises N second process data $T_j$, and $e_1$, $e_2$, and $e_3$ are all preset high-pass filtering coefficients;

Here, the baseline of the entire signal is pulled to the same horizontal line to the maximum extend by adjusting the positions of relative baselines of every two adjacent data points of the second-class PPG original signal;

Step 223, a maximum value is extracted from the second process sequence $(T_1, T_2...T_j...T_N)$ to generate a maximum reference value max, and a minimum value is extracted from the second process sequence $(T_1, T_2...T_j...T_N)$ to generate a minimum reference value min;

Step 224, normalized filtering is performed on the second process sequence $(T_1, T_2...T_j...T_n)$ to generate a third process sequence $(P_1, P_2...P_j...P_N)$ specifically by setting $P_j$ according to a formula $P_j = \dfrac{T_j - min}{max - min}$ ;

Wherein, the third process sequence $(P_1, P_2...P_j...P_N)$ comprises N third process data $P_j$;

Here, after baseline drift removal is performed on the second-class PPG original signal, normalized filtering is performed on the second process sequence $(T_1, T_2...T_j...T_N)$ according to a ratio of the amplitude of each signal point to a full signal maximum amplitude, and the signal shape and amplitude after filtering are shown in FIG. 2 which is a schematic diagram of the PPG signal before and after filtering in this embodiment of the invention;

Step 225, the standard PPG data sequence is set as the third process sequence $(P_1, P_2...P_j...P_N)$, and Step 3 is performed;

Step 23, when the data source identifier is the third-class PPG video identifier, video quality detection and normalized signal conversion is performed on the third-class PPG video data to generate the standard PPG data sequence;

Step 23 comprises: Step 231, when the data source identifier is the third-class PPG video identifier, video data frame image extraction is performed on the third-class PPG video data to generate a third-class PPG video frame image sequence;

Wherein, the third-class PPG video frame image sequence comprises multiple third-class PPG video frame images;

Here, the third-class PPG video data is a file of a common standard video format, and image frames can be extracted from the video file in seconds through standard video processing software or a standard video processing method. For example, if the length of a video is 5s and each second of the video includes 24 frames of images, the extracted third-class PPG video frame image sequence comprises 5*24=120 third-class PPG video frame image vector data (120 images);

Step 232, one-dimensional red light signal extraction is performed on all the third-class PPG video frame images in the third-class PPG video frame image sequence according to a preset red light pixel threshold range to generate a first red light digital signal, and one-dimensional green light signal extraction is performed on all the third-class PPG video frame images in the third-class PPG video frame image sequence according to a preset green light pixel threshold range to generate a first green light digital signal;

Step 232 comprises: Step 2321, the first red light digital signal is initialized to be null, the first green light digital signal is initialized to be null, a first index is initialized to 1, and a first total number is initialized to a total number of the third-class PPG video frame images in the third-class PPG video frame image sequence;

Step 2322, a first-index frame image is set as the third-class PPG video frame image, corresponding to the first index, in the third-class PPG video frame image sequence;

Step 2323, all pixels, meeting the red light pixel threshold range, in the first-index frame image are collected to generate a red pixel set, a total number of the pixels in the red pixel set is calculated to generate a red pixel total number, the sum of pixel values of all the pixels in the red pixel set is calculated to generate a red pixel value sum, and first-index frame red light channel data is generated according to a quotient obtained by dividing the sum of red pixel values by the total number of red pixel; and signal points are added into the first red light digital signal using the first-index frame red light channel data as signal point data;

For example, the total number of all pixels, meeting the red light pixel threshold range, in a first frame of image are extracted to generate a total number of red pixel (because the internal structures or blood vessels at different positions may reflect and transmit light to different degrees, the light transmittances will be different, the shades of red pixels in the recorded video will also be different, so the pixel threshold range is adopted), and the sum of pixel values of all the pixels, meeting the red light pixel threshold range, in the first frame of image is calculated to generate a sum of red pixel values, and the first-index frame red channel data = the red pixel value sum / the red pixel total number;

Step 2324, all pixels, meeting the green light pixel threshold range, in the first-index frame image are collected to generate a green pixel set, a total number of the pixels in the green pixel set is calculated to generate a total number of green pixels, the sum of pixel values of all the pixels in the green pixel set is calculated to generate a green pixel value sum, and first-index frame green light channel data is generated according to a quotient obtained by dividing the sum of green pixel values by the total number of green pixels; and signal points are added into the first green light digital signal using the first-index frame green light channel data as signal point data;

For example, the total number of all pixels, meeting the green light pixel threshold range, in a first frame of image are extracted to generate a total number of green pixel (because the internal structures or blood vessels at different positions may reflect and transmit light to different degrees, the light transmittances will be different, the shades of green pixels in the recorded video will also be different, so the pixel threshold range is adopted), and the sum of pixel values of all the pixels, meeting the green light pixel threshold range, in the first frame of image is calculated to generate a sum of green pixel value, and the first-index frame green channel data = the sum of green pixel value / the total number of green pixel;

Step 2325, the first index is increased by 1;

Step 2326, whether the first index is greater than the first total number is determined; if the first index is less than or equal to the first total number, Step 2322 is performed; or, if the first index is greater than the first total number, Step 233 is performed;

Here, in Step 232, two types of light channel data, red light channel data and green light channel data, are extracted from all the third-class PPG video frame images in the third-class PPG video frame image sequence in the following way: weighted average calculation is performed on specific pixels in each frame image to obtain a pixel average that is used to represent the color channel data of the corresponding light in the frame image; and all frame images in the video are processed in the same way in chronological order to obtain two segments of one-dimensional digital signals: first red light digital signal and first green light digital signal.

Step 233, according to a preset band-pass filtering frequency threshold range, band-pass filtering preprocessing is performed on the first red light digital signal to generate a second red light digital signal, and band-pass filtering preprocessing is performed on the first green light digital signal to generate a second green light digital signal;

Here, signal filtering preprocessing, namely denoising, is performed on the two types of light channel data. In Embodiment 1, band-pass filtering is used for denoising, that is, a band-pass filtering frequency threshold range is preset, and signals, interference and noise lower or higher than the band-pass filtering frequency threshold range are restrained based on the band-pass filtering principle. Generally, the band-pass filtering frequency threshold range is 0.5-10 THz. When some mobile terminals are used for band-pass filtering, a finite impulse response (FIR) filtering module is used;

Step 234, maximum frequency difference determination is performed on the second red light digital signal and the second green light digital signal to generate a first determination result;

Step 234 comprises: Step 2341, digital signal time domain-frequency domain conversion is performed on the second red light digital signal through discrete Fourier transform to generate a red light frequency domain signal, and digital signal time domain-frequency domain conversion is performed on the second green light digital signal through discrete Fourier transform to generate a green light frequency domain signal;

Step 2342, a maximum-energy frequency is extracted from the red light frequency domain signal to generate a maximum red light frequency, and a maximum-energy frequency is extracted from the green light frequency domain signal to generate a maximum green light frequency;

Step 2343, a frequency difference between the maximum red light frequency and the maximum green light frequency is calculated to generate a maximum red-green frequency difference;

Step 2344, when the maximum red-green frequency difference does not exceed a preset maximum frequency difference threshold range, the first determination result is set as the up-to-standard signal identifier;

Here, in Step 234, frequency domain signals of the second red light digital signal and the second green light digital signal are obtained through discrete Fourier transform; maximum-energy frequencies are obtained according to the frequency domain signals (generally, this frequency corresponds to the heart rate); whether the maximum-energy frequencies of the two digital signals are consistent is checked; if an error is within an allowable error range, the first determination result is set as the up-to-standard signal identifier; or, if the error is large, the first determination result is set as a not-up-to-standard signal identifier;

Step 235, when the first determination result is the up-to-standard signal identifier, signal-to-noise ratio determination is performed on the second red light digital signal and the second green light digital signal to generate a second determination result;

Step 235 comprises: Step 2351, when the first determination result is the up-to-standard signal identifier, according to a preset band-stop filtering frequency threshold range, valid signal points, meeting the band-stop filtering frequency threshold range, are removed from the second red light digital signal through multi-order Butterworth band-stop filtering to generate a red light noise signal, and valid signal points, meeting the band-stop filtering frequency threshold range, are removed from the second green light digital signal through multi-order Butterworth band-stop filtering to generate a green light noise signal;

Step 2352, signal energy of the second red light digital signal is calculated to generate red light signal energy, signal energy of the red light noise signal is calculated to generate red light noise energy, valid red light signal energy is generated according to a difference between the red light signal energy and the red light noise energy, and a red light signal-to-noise ratio is generated according to a ratio of the valid red light signal energy to the red light noise energy;

Step 2353, signal energy of the second green light digital signal is calculated to generate green light signal energy, signal energy of the green light noise signal is calculated to generate green light noise energy, valid green light signal energy is generated according to a difference between the green light signal energy and the green light noise energy, and a green light signal-to-noise ratio is generated according to a ratio of the valid green light signal energy to the green light noise energy;

Step 2354, when any one of the red light signal-to-noise ratio and the green light signal-to-noise ratio is greater than or equal to a signal-to-noise threshold, the second determination result is set as the up-to-standard signal identifier;

Here, in Step 235, secondary filtering is performed on red and green lights: the secondary filtering is band-stop filtering, that is, signals within the band-stop filtering frequency threshold range are restrained specifically through multi-order Butterworth band-stop filtering (such as, four-order Butterworth band-stop filtering or one-order Butterworth band-stop filtering); through band-stop filtering, noise and interference signals are reserved to generate noise signals, and then valid signals and the noise signals are calculated to generate signal-to-noise ratios; and finally, whether the red and green light digital signals are up to standard are recognized according to the signal-to-noise ratios;

Step 236, when the second determination result is the up-to-standard signal identifier, normalized PPG signal data sequence generation is performed on the second red light digital signal and the second green light digital signal to generate the standard PPG data sequence;

Here, because a standard PPG data sequence is of a regressive data structure while the value of color channel data of the second red light digital signal and the second green light digital signal is greater than 1, normalization processing should be performed on the second red light digital signal and the second green light digital signal. Many normalization methods can be used for normalization processing, and will not be further expounded in this embodiment;

Step 236 comprises: when the second determination result is the up-to-standard signal identifier, signal data normalization processing is performed on the second red light digital signal and the second green light digital signal respectively to generate a normalized red light signal and a normalized green light signal; a red light data sequence of the standard PPG data sequence is set as the normalized red light signal, and a green data sequence of the standard PPG data sequence is set as the normalized green light signal, wherein the standard PPG data sequence comprises the red light data sequence and the green light data sequence.

Here, due to the number of lights in a video, the standard PPG data sequence generated according to the third-class video data differs from the standard PPG data sequences generated according to the first-class original signal and the second-class PPG original signal in the following aspect: the standard PPG data sequences generated according to the first-class original signal and the second-class PPG original signal are always one-channel data; if a single light exists in the video, the standard PPG data sequence extracted from the third-class video data is single-channel data; if red-green lights exist in the video, the standard PPG data sequence extracted from the third-class video data is double-channel data.

Step 3, a CNN model identifier is acquired;

Wherein, the CNN model identifier is a first-class CNN identifier or a second-class CNN identifier;

Here, CNN models will be introduced briefly. The CNN has always been one of the key algorithms in the field of feature recognition. When applied to image recognition, the CNN is used, during fine classification and recognition, to extract discriminant features of images, which are then learnt by other classifiers. When applied to the field of blood pressure feature recognition, the CNN is used to perform PPG signal feature extraction and calculation on an input one-dimensional standard PPG data sequence: after convolution and pooling are performed on the input standard PPG data sequence, feature data in conformity to PPG signal features are reserved for a fully connected layer to perform regression calculation. This embodiment of the invention provides two types of CNN models to perform blood pressure prediction on the standard PPG data sequence: first-class CNN model and second-class CNN model. The CNN model identifier is used to distinguish and recognize these two CNN models, thus being the first-class CNN identifier or the second-class CNN identifier;

First, these two CNN models are different in feature extraction object: the first-class CNN model performs feature extraction directly on the standard PPG data sequence according to the time-domain amplitude of signals, and the second-class CNN model converts the standard PPG data sequence into a time-domain graph data sequence and then performs on the time-domain graph data sequence;

Second, these two CNN models are different in internal network structure:

(1) The first-class CNN model comprises multiple CNN network layers and a fully connected layer, and each CNN network layer comprises a convolutional layer and a pooling layer; the second-class CNN model comprises a two-dimensional convolutional layer, a maximum pooling layer, a batch normalization layer, an activation layer, an add layer, a global average pooling layer, a dropout layer and a fully connected layer; the first-class CNN model is a CNN model that has been trained through blood pressure feature extraction, and is specifically composed of multiple CNN network layers and a fully connected layer, and each CNN network layer comprises a convolutional layer used to perform blood pressure feature extraction and calculation on input data of the CNN model and a pooling layer used to perform down-sampling on an extraction result of the convolutional layer, a preset convolutional layer number threshold indicates the specific number of the CNN network layers of the CNN model, and an output result of each CNN network layer is used as an input of the next CNN network layer; and finally, a result obtained after the preset convolutional layer number threshold times of calculation by the CNN network layers is input to the fully connected layer of the CNN model for regression calculation;

(2) Different from the first-class CNN model, the second-class CNN model adopts a customized convolutional network structure, and comprises a two-dimensional convolutional layer, a maximum pooling layer, a batch normalization layer, an activation layer, an add layer, a global average pooling layer, a dropout layer and a fully connected layer, wherein the two-dimensional convolutional layer may comprise multiple sub-convolutional layers and is used to perform multiple times of convolution calculation on input data, and a convolution result (four-dimensional tensor) output the two-dimensional convolutional layer comprises multiple one-dimensional tensor; the maximum pooling layer is used to sampling the convolution result by acquiring a maximum value of each one-dimensional vector to reduce the data size; the batch normalization layer is used to perform data normalization on an output result of the maximum pooling layer; the activation layer performs neural network connection on an output result of the batch normalization layer by means of a nonlinear activation function; the add layer is used to perform weighed sum calculation on an output result of the activation layer; the global average pooling layer is used to perform weighted average calculation on an output result of the add layer; the dropout layer is used to clip an output result of the global average pooling layer; and finally, the fully connected layer performs dichotomous regression calculation on a clipped output result of the dropout layer to output regression calculation results of the diastolic pressure and the systolic pressure.

Step 4, a corresponding CNN model is selected to perform blood pressure prediction on the standard PPG data sequence according to the CNN model identifier;

Step 4 comprises: Step 41, when the CNN model identifier is the first-class CNN identifier, the first-class CNN model is selected to perform blood pressure prediction on the standard PPG data sequence;

Step 41 comprises: Step 411, first-class CNN model input data conversion is performed on the standard PPG data sequence according to a preset first-class CNN input width threshold to generate an input data four-dimensional tensor;

Here, the first-class CNN input width threshold is the maximum value of an initial input data length of the first-class CNN model; and in this embodiment of the invention, input data of the first-class CNN model is of a four-dimensional tensor format;

Step 412, according to a preset convolutional layer number threshold, multilayer convolution and pooling calculation is performed on the input data of four-dimensional tensor by means of the CNN network layers of the first-class CNN model to generate a feature data of four-dimensional sensor;

Here, a preprocessed input data of four-dimensional tensor is input to the CNN network layer of the trained first-class CNN model to perform feature extraction to generate the feature data of four-dimensional sensor, of which the data format is also the four-dimensional tensor format; as described above, the CNN network layer is composed of multiple convolutional layers and multiple pooling layers; generally, one convolutional layer matched one pooling layer and is then connected to the next convolutional layer, and the final layer number depends on the threshold of convolutional layer number, for example, a network comprising four convolutional layers and four pooling layer is called a four-layer convolution network; the convolutional layers perform convolution calculation to convert an input into outputs of different dimensions, and these output may be regarded as another presentation of the input; and the pooling layers are used to output a quantity to simplify the operation and promote the network to extract more valid information;

Step 413, two-dimensional matrix construction of fully connected layer input data two-dimensional matrix construction of fully connected layer input data is performed according to the four-dimensional tensor of feature data to generate an two-dimensional matrix of input data, and feature data regression calculation is performed on the two-dimensional matrix of input data by means of the fully connected layer of the first-class CNN model to generate a two-dimensional matrix of blood pressure regression data of blood pressure regression data;

Here, input and output data of the first-class CNN model is of a two-dimensional matrix format, so before regression calculation is performed by the fully connected layer, dimension reduction needs to be performed on the four-dimensional tensor output by the CNN network layer to convert the four-dimensional tensor into a two-dimensional matrix; the fully connected layer of the first-class CNN model is composed of multiple sub-fully connected layers, each node of each sub-fully connected layer is connected to all nodes of the prior sub-fully connected layer to integrate all features extracted previously, and the number of nodes and an activation function (generally the ReLU function, or other functions) of each sub-fully connected layer may be set; and the number of nodes of the last sub-fully connected layer is set to 2, so that two regression calculation values, that respectively represent the systolic pressure and the diastolic pressure of the blood pressure, can be obtained after several layers of fully connected calculation;

Step 414, a preset prediction mode identifier is acquired;

Wherein, the prediction mode identifier is a mean prediction identifier or a dynamic prediction identifier;

Here, the prediction mode identifier is a system variable, and output contents may be further predicted by means of the variable according to blood pressure prediction values obtained after regression calculation of the fully connected layer: when the prediction mode identifier is a mean prediction identifier, it indicates that mean blood pressure data in the original signal needs to be output; or, when the prediction mode identifier is a dynamic prediction identifier, it indicates that a blood pressure change data sequence within the time period of the original signal needs to be output;

Step 415, when the prediction mode identifier is the mean prediction identifier, mean blood pressure calculation is performed on the two-dimensional matrix of blood pressure regression data to generate a mean blood pressure prediction data pair;

Wherein, the mean blood pressure prediction data pair comprises mean systolic pressure prediction data and mean diastolic pressure prediction data;

Here, the two-dimensional matrix of blood pressure regression data of blood pressure regression data may be construed as a vector sequence comprising multiple one-dimensional vectors [2], and the mean of smaller values of each one-dimensional vectors [2] is calculated to obtain mean diastolic pressure prediction data (that the smaller value is calculated is because the systolic pressure is greater than the diastolic pressure, the smaller one of two regression calculation values is a predicted value of the diastolic pressure), the mean of larger values in the one-dimensional vectors [2] is calculated to obtain mean systolic pressure prediction data (that the larger value is calculated is because the systolic pressure is greater than the diastolic pressure, the larger one of the two regression calculation values is a predicted value of the systolic pressure);

Step 416, when the prediction mode identifier is the dynamic prediction identifier, dynamic blood pressure data extraction is performed on the two-dimensional matrix of blood pressure regression data of blood pressure regression data to generate a one-dimensional data sequence of one-dimensional data sequence of dynamic blood pressure prediction ;

Here, the systolic pressure and diastolic pressure in all the one-dimensional vectors [2] in the two-dimensional matrix of blood pressure regression data of blood pressure regression data are extracted to form a data sequence, and the dynamic change of the blood pressure within a period of time is reflected by the data sequence;

Step 42, when the CNN model identifier is the second-class CNN identifier, the second-class CNN model is selected to perform wavelet transform-based blood pressure prediction on the standard PPG data sequence.

Here, as described above, the second-class CNN model is used to perform feature extraction on a time-domain graph data sequence, so it is necessary to convert the standard PPG data sequence, which is a time-domain data sequence, into a time-frequency data sequence and then convert the time-frequency data sequence into a time-domain graph data sequence before the second-class CNN model is used; conventionally, time-frequency conversion of signals is realized through Fourier transform, but due to the fact that the size of the time-frequency analysis window for Fourier transform is fixed, feature data may be lost when Fourier transform is used to process non-stationary PPG signals; in this embodiment of the invention, wavelet transform, which is a time-frequency analysis method based on Fourier transform and can highlight local features of signals in principle, is used to realize time domain-frequency domain conversion; in this embodiment, continuous wavelet transform (one method of wavelet transform) is used for conversion of the PPG signal; and a common method used for converting a time-frequency data sequence into a time-domain graph data sequence is to use a red-green-blue (RGB) mode.

Step 42 comprises: Step 421, data fragment division is performed on the standard PPG data sequence to generate standard PPG data fragments

Step 422, a preset wavelet basis type, a scalability factor array and a mobile factor array are acquired;

Wherein, the scalability factor array comprises H scalability factors, the mobile factor array comprises L mobile factors, and H and L are both integers;

Compared with short-time Fourier transform, continuous wavelet transform, as an important means for local analysis of signals, has an adjustable window, thus having a high capacity to analyze non-stationary signals; the signals can be refined on multiple scales through a scaling and translational operation of wavelets, high-frequency components of the signals may have a high time resolution, and low-frequency components of the signals may have a high frequency resolution; continuous wavelet transform has three key parameters: wavelet basis, scalability factor and mobile factor, wherein the wavelet basis type is a wavelet function specifically used for wavelet transform, the scalability factor is a scale parameter that may change automatically in the wavelet transform process, and the mobile factor is a mobile time parameter that may change automatically in the wavelet transform process;

Step 423, signal decomposition is performed on the standard PPG data fragments through continuous wavelet transform according to the scalability factors in the scalability factor array, the mobile factors in the scalability factor array and the wavelet basis type to generate a PPG wavelet coefficient matrix [H, L];

Here, the PPG wavelet coefficient matrix [H, L] is formed by H*L wavelet coefficients, and each wavelet coefficient is a complex number that reflects the scalability factor and the mobile factor;

Step 424, the PPG wavelet coefficient matrix is transformed into a real matrix through a modulo operation on matrix elements, and normalization processing is performed on values of matrix elements in the real matrix to generate a PPG normalized matrix [H, L];

Here, a complex matrix is transformed into a real matrix through a modulo operation, and values of all matrix elements in the real matrix are normalized to obtain a PPG normalized matrix; if the PPG normalized matrix [H, L] is construed as a data sequence, this data sequence is a time-frequency data sequence obtained after time domain-frequency domain conversion is performed on the standard PPG data sequence;

Step 425, an RGB color palette matrix is acquired, and PPG time-frequency tensor conversion is performed on the PPG normalized matrix [H, L] according to the RGB color palette matrix to generate a PPG time-frequency three-dimensional tensor [H, L, 3];

The RGB mode, as a color standard in the industry, is used to obtain various colors by changing three color channels, red (R), green (G) and blue (B) and superposing of these three colors, and this standard includes almost all colors that can be perceived by human eyes and is one of the most widely used color systems; assume the RGB color palette matrix comprises 256 color vectors, each color vector has a length of 3 and comprises values of the three primary colors;

The values of all the matrix elements in the PPG normalized matrix [H, L] are within 0-1; when PPG time-frequency tensor conversion is performed on the PPG normalized matrix [H, L], and the range 0-1 is divided into 256 segments; then, all the elements in the PPG normalized matrix [H, L] are polled to turn original values of the elements into indexes of the segments to which the values of the elements belong (for example, the first segment is 0-1/256 and the value of one element is 1/257, the value 1/257 of this element will be turned into 1; the 256th segment is 255/256-1 and the value of one element is 511/512, the value 511/512 of this element will be turned into 256); and finally, the values of the elements in the PPG normalized matrix [H, L] are turned from 0-1 to 1-256;

Assume the RGB color palette matrix comprises 256 colors, each element in the PPG normalized matrix [H, L] corresponds to one RGB color vector in the RGB color palette matrix (each color vector is a one-dimensional vector [3] comprising the pixel values of the red color, the green color and the blue color), and this RGB color vectors [3] is extracted from the RGB color palette matrix and is then added to a corresponding position of the PPG normalized matrix [H, L] to generate a time-frequency graph sequence, namely the PPG time-frequency three-dimensional tensor [H, L, 3];

Step 426, according to a preset second-class CNN input width threshold, tensor shape reconstruction is performed on the PPG time-frequency three-dimensional tensor [H, L, 3] through a bicubic interpolation algorithm to generate a PPG convolutional three-dimensional tensor [K, K, 3];

Wherein, K is the second-class CNN input width threshold;

Here, the size of the PPG time-frequency three-dimensional tensor [H, L, 3] may not meet the requirement for the input size of the second-class CNN model; when the size of the PPG time-frequency three-dimensional tensor [H, L, 3] is smaller than the input size of the second-class CNN model, a bicubic interpolation algorithm (a method for increasing the number of matrix points in matrix data by interpolation calculation, generally, the interpolation technique is used to increase the graphic data and the graphic size) is used to add mean values to change the shape of the three-dimensional tensor and increase the time-frequency graphic size, and finally, the PPG convolutional three-dimensional tensor [K, K, 3] meeting the requirement of the second-class CNN model is generated;

Step 427, blood pressure prediction is performed on the PPG convolutional three-dimensional tensor [K, K, 3] using the second-class CNN model to generate a PPG blood pressure prediction data pair;

Wherein, the PPG blood pressure prediction data pair comprises PPG systolic pressure prediction data and PPG diastolic pressure prediction data.

Here, the second-class CNN model in this embodiment comprises: a two-dimensional convolutional layer, a maximum pooling layer, a batch normalization layer, an activation layer, an add layer, a global average pooling layer, a dropout layer and a fully connected layer, wherein the two-dimensional convolutional layer may comprise multiple sub-convolutional layers and is used to perform multiple times of convolution calculation on input data, and a convolution result (four-dimensional tensor) output by the two-dimensional convolutional layer comprises multiple one-dimensional tensor; the maximum pooling layer is used to sampling the convolution result by acquiring a maximum value of each one-dimensional vector to reduce the data size; the batch normalization layer is used to perform data normalization on an output result of the maximum pooling layer; the activation layer performs neural network connection on an output result of the batch normalization layer by means of a nonlinear activation function; the add layer is used to perform weighed sum calculation on an output result of the activation layer; the global average pooling layer is used to perform weighted average calculation on an output result of the add layer; the dropout layer is used to clip an output result of the global average pooling layer; and finally, the fully connected layer performs dichotomous regression calculation on a clipped output result of the dropout layer to output regression calculation results of PPG systolic pressure prediction data and PPG diastolic pressure prediction data.

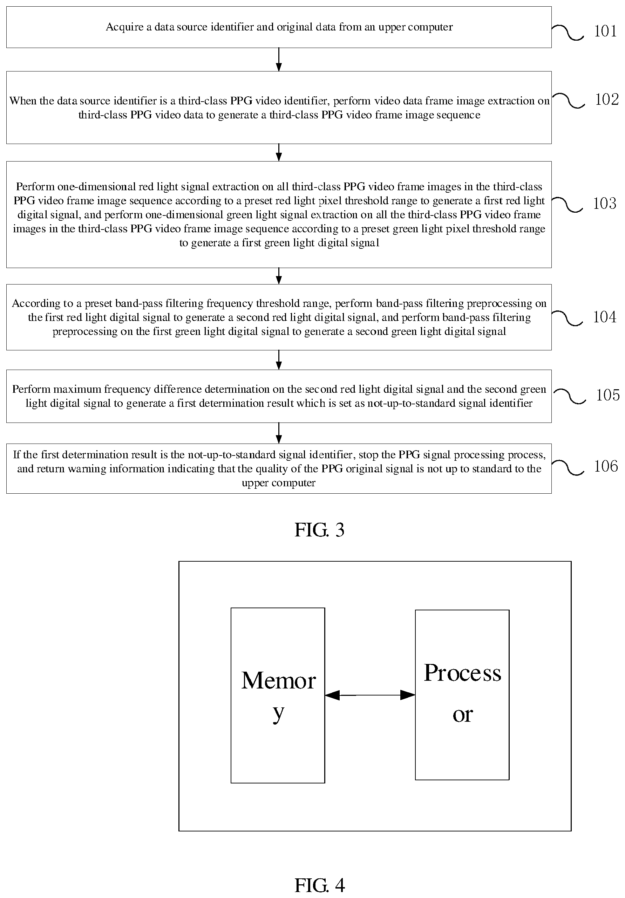

As shown in FIG. 3 which is a schematic diagram of a method for performing video quality detection on third-class PPG video data according to Embodiment 2 of the invention, the method mainly comprises the following steps:

Step 101, a data source identifier and original data are acquired from an upper computer;

Wherein, the data source identifier is one of a first-class PPG original signal identifier, a second-class PPG original signal identifier and a third-class PPG video identifier; and corresponding the data source identifier, the original data is one of a first-class PPG original data, a second-class PPG original signal and third-class PPG video data.

Here, to guarantee the compatibility with various PPG data source acquisition approaches, the data source identifier is set to distinguish the type of acquired original data: a first-class PPG original data, a second-class PPG original signal ; as shown in FIG. 2 which is a schematic diagram of PPG signals before and after filtering according to this embodiment of the invention, the first-class PPG original signal and the second-class PPG original signal are generally acquired from the skin surface of a test subject through a PPG signal acquisition device, wherein signals with a non-obvious horizontal baseline drift are classified as first-class PPG original signals, and signals with an obvious horizontal baseline drift are classified as second-class PPG original signals. Generally, the third-class PPG video data is obtained by photographing the skin surface of the test subject through a video recording device and is of a common video format.

Step 102, when the data source identifier is the third-class PPG video identifier, video data frame image extraction is performed on the third-class PPG video data to generate a third-class PPG video frame image sequence;

Wherein, the third-class PPG video frame image sequence comprises multiple third-class PPG video frame images; Here, the third-class PPG video data is a file of a common standard video format, and image frames can be extracted from the video file in seconds through standard video processing software or a standard video processing method. For example, if the length of a video is 5s and each second of the video includes 24 frames of images, the extracted third-class PPG video frame image sequence comprises 5*24=120 third-class PPG video frame image vector data (120 images);

Step 103, one-dimensional red light signal extraction is performed on all the third-class PPG video frame images in the third-class PPG video frame image sequence according to a preset red light pixel threshold range to generate a first red light digital signal, and one-dimensional green light signal extraction is performed on all the third-class PPG video frame images in the third-class PPG video frame image sequence according to a preset green light pixel threshold range to generate a first green light digital signal;

Here, information of two lights, red light and green light, is extracted from all the third-class PPG video frame images in the third-class PPG video frame image sequence in the following way: weighted average calculation is performed on specific pixels in each frame image to obtain a pixel average that is used to represent the color channel data of the corresponding light in the frame image; and all frame images in the video are processed in the same way in chronological order to obtain two segments of one-dimensional digital signals: first red light digital signal and first green light digital signal.

Step 104, according to a preset band-pass filtering frequency threshold range, band-pass filtering preprocessing is performed on the first red light digital signal to generate a second red light digital signal, and band-pass filtering preprocessing is performed on the first green light digital signal to generate a second green light digital signal;

Here, signal filtering preprocessing, namely denoising, is performed on the digital signals of the two lights extracted from the video data. In Embodiment 1, band-pass filtering is used for denoising, that is, a band-pass filtering frequency threshold range is preset, and signals, interference and noise lower or higher than the band-pass filtering frequency threshold range are restrained based on the band-pass filtering principle. Generally, the band-pass filtering frequency threshold range is 0.5-10 THz. When some mobile terminals are used for band-pass filtering, a finite impulse response (FIR) filtering module is used;

Step 105, maximum frequency difference determination is performed on the second red light digital signal and the second green light digital signal to generate a first determination result as not up to standard identifier;

Here, frequency domain signals of the second red light digital signal and the second green light digital signal are obtained through discrete Fourier transform; maximum-energy frequencies are obtained according to the frequency domain signals (generally, this frequency corresponds to the heart rate); whether the maximum-energy frequencies of the two digital signals are consistent is checked; if an error is within an allowable error range, the first determination result is set as an up-to-standard signal identifier; or, if the error is large, the first determination result is set as a not-up-to-standard signal identifier.

Step 106, if the first determination result is the not-up-to-standard signal identifier, the PPG signal processing process is stopped, and warning information indicating that the quality of the PPG original signal is not up to standard is returned to the upper computer.

Here, this error may be caused by many reasons. For example, due to a large distance between the skin surface of a test subject and a photographing device during the video recording process, light leaking occurs, so there may be a large deviation in the red channel data or the green channel data extracted from the video frame images, which makes the frequency difference therebetween exceed a preset range; once the video quality is not up to standard, blood pressure data deduced from the video data will not be accurate and may even incorrect, so the analysis of the video data should be stopped, and an upper application will mark the video data as unqualified when receiving the warning information indicating that the quality of the PPG original signal is not up to standard and may further initiate a re-photographing operation.

As shown in FIG. 4 which is a schematic diagram of a blood pressure prediction device provided by Embodiment 3 of the invention. Equipment comprises: a processor and a memory. The memory may be connected to the process through a bus. The memory may be a nonvolatile memory such as a hard disk drive or a flash memory, and a software program and an equipment drive program are stored in the memory. The software program can implement all functions of the method provided by the embodiments of the invention, and the equipment drive program may be a network and interface drive program. The processor is used to execute the software program, and when the software program is executed, the method provided by the embodiments of the invention is implemented.

It should be noted that the embodiments of the invention further provide a computer-readable storage medium having a computer program stored therein, and when the computer program is executed by a processor, the method provided by the embodiments of the invention is implemented.

The embodiments of the invention further provide a computer program product comprising instructions. When the computer program product runs on a computer, a processor implements the method mentioned above.

According to the blood pressure prediction method and device using multiple data sources provided by the embodiments of the invention, two signal filtering and shaping methods are provided for directly acquired PPG signals, and a video quality detection and normalized signal conversion method is provided for indirectly generated PPG signal, and a uniform standard PPG data sequence is finally generated for blood pressure prediction; and the embodiments of the invention provide two optional CNN models for blood pressure prediction. By adoption of the method and device provided by the embodiments of the invention, the capacity to process various PPG signal data sources and the capacity to manage various blood pressure prediction models of an application are improved, and the compatibility with various data sources for blood pressure prediction of the application is improved.

Those skilled should further appreciate that the units and arithmetic steps described in conjunction with the embodiments in this specification may be implemented by electronic hardware, computer software, or a combination of these two. To clearly explain the interchangeability of hardware and software, the components and steps of illustrative embodiments have been generally described according to their functions. Whether these functions are implemented by hardware or software depends on specific applications and design constraints of the technical solutions. For each specific application, those skilled may implement these functions in different ways, which should not be construed as exceeding the scope of the invention.

The steps of the method or algorithm described in the embodiments in this specification may be implemented by hardware, software modules executed by a processor, or a combination of these two. The software modules may be configured in a random access memory (RAM), a memory, a read-only memory (ROM), an electrically programmable ROM, an electrically erasable and programmable ROM, a register, a hard disk, a removable disk, a CD-ROM, or a storage medium in any other forms in the art.

The purposes, technical solutions and beneficial effects of the invention are described in further detail with reference to the above specific implementations. It should be understood that the above implementations are merely specific ones of the invention, and are not used to limit the protection scope of the invention, which is defined by the claims.

## Claims

1. A blood pressure prediction method using multiple data sources, comprising:

acquiring a data source identifier and original data from an upper computer (1, 101), wherein the data source identifier is one of a first-class PPG original signal identifier, a second-class PPG original signal identifier and a third-class PPG video identifier; and the original data is one of a first-class PPG original data, a second-class PPG original signal and third-class PPG video data, and corresponds to the data source identifier; which includes:

the first-class PPG original signal and the second-class PPG original signal are acquired from the skin surface of a test subject through a PPG signal acquisition device, wherein signals with a non-obvious horizontal baseline drift are classified as first-class PPG original signals, and signals with an obvious horizontal baseline drift are classified as second-class PPG original signals, wherein the third-class PPG video data is obtained by photographing the skin surface of the test subject through a video recording device;
wherein the data source identifier is set to distinguish the type of acquired original data, wherein the first-class PPG identifier corresponds to a first-class PPG original signal, the second-class PPG identifier corresponds to a second-class PPG original signal, and the third-class PPG identifier corresponds to third-class PPG video data;
preprocessing the original data according to the data source identifier (2); when the data source identifier is the first-class PPG original signal identifier, performing normalized filtering on the first-class PPG original signal to generate a standard PPG data sequence;
when the data source identifier is the second-class PPG original signal identifier, performing baseline drift removal and normalized filtering on the second-class PPG original signal to generate the standard PPG data sequence; and when the data source identifier is the third-class PPG video identifier, performing video quality detection and normalized signal conversion on the third-class PPG video data to generate the standard PPG data sequence;
acquiring a CNN model identifier (3), wherein the CNN model identifier includes a first-class CNN identifier or a second-class CNN identifier; and
selecting a corresponding CNN model to perform blood pressure prediction on the standard PPG data sequence according to the CNN model identifier (4); when the CNN model identifier is the first-class CNN identifier, selecting a first-class CNN model to perform blood pressure prediction on the standard PPG data sequence; or when the CNN model identifier is the second-class CNN identifier, selecting a second-class CNN model to perform wavelet transform-based blood pressure prediction on the standard PPG data sequence;
**characterized in that**
when the data source identifier is the third-class PPG video identifier, performing video quality detection and normalized signal conversion on the third-class PPG video data to generate the standard PPG data sequence, comprises:

when the data source identifier is the third-class PPG video identifier, performing video data frame image extraction on the third-class PPG video data to generate a third-class PPG video frame image sequence (102), wherein the third-class PPG video frame image sequence comprises multiple third-class PPG video frame images;
performing one-dimensional red light signal extraction on all the third-class PPG video frame images in the third-class PPG video frame image sequence according to a preset red light pixel threshold range to generate a first red light digital signal, and performing one-dimensional green light signal extraction on all the third-class PPG video frame images in the third-class PPG video frame image sequence according to a preset green light pixel threshold range to generate a first green light digital signal (103);

according to a preset band-pass filtering frequency threshold range, performing band-pass filtering preprocessing on the first red light digital signal to generate a second red light digital signal, and performing band-pass filtering preprocessing on the first green light digital signal to generate a second green light digital signal (104);

performing maximum frequency difference determination on the second red light digital signal and the second green light digital signal to generate a first determination result (105);

when the first determination result is an up-to-standard signal identifier, performing signal-to-noise ratio determination on the second red light digital signal and the second green light digital signal to generate a second determination result; and

when the second determination result is the up-to-standard signal identifier, performing normalized PPG signal data sequence generation on the second red light digital signal and the second green light digital signal to generate the standard PPG data sequence,

wherein when the data source identifier is the first-class PPG original signal identifier, performing normalized filtering on the first-class PPG original signal to generate a standard PPG data sequence, comprises:

when the data source identifier is the first-class PPG original signal identifier,

performing data sampling on the first-class PPG original signal according to a preset first-class signal sampling threshold to generate a first-class PPG sampling data sequence $(X_1, X_2...X_i...X_M)$, wherein the first-class PPG sampling data sequence $(X_1, X_2...X_i...X_M)$ comprises M first-class PPG sampling data $X_i$, M is an integer, and i ranges from 1 to M;

performing normalized filtering on the first-class PPG sampling data sequence $(X_1, X_2...X_i...X_M)$ to generate a first process sequence $(Y_1, Y_2...Y_i...Y_M)$; when i is 1, setting $Y_i = X_i$; or, when i is greater than 1, setting $Y_i$ according to a formula

$$Y_i = \frac{a \times Y_{i-1}}{b} + \frac{X_i}{b \times c}$$

wherein the first process sequence $(Y_1, Y_2...Y_i...Y_M)$ comprises M first process data $Y_i$, a and b are preset first-class filtering constants, and c is a gain coefficient of the first-class PPG original signal; and

setting the standard PPG data sequence as the first process sequence $(Y_1, Y_2...Y_i...Y_M)$.

2. The blood pressure prediction method using multiple data sources according to Claim 1, wherein when the data source identifier is the second-class PPG original signal identifier, performing baseline drift removal and normalized filtering on the second-class PPG original signal to generate the standard PPG data sequence, comprises:

when the data source identifier is the second-class PPG original signal identifier, performing data sampling on the second-class PPG original signal according to a preset second-class signal sampling threshold to generate a second-class PPG sampling data sequence $(S_1, S_2...S_j...S_N)$, wherein the second-class PPG sampling data sequence $(S_1, S_2...S_j...S_N)$ comprises N second-class PPG sampling data Sj, N is an integer, and j ranges from 1 to N;

performing baseline drift removal and filtering on the second-class PPG sampling data sequence $(S_1, S_2...S_j...S_N)$ to generate a second process sequence $(T_1, T_2...T_j...T_N)$; when j is 1, setting

$$T_j = S_j$$

or, when j is greater than 1, setting Tj according to a formula

$$T_j = e_1 \times S_j + e_2 \times S_{j-1} - e_3 \times T_{j-1}$$

wherein the second process sequence $(T_1, T_2...T_j...T_N)$ comprises N second process data $T_j$, and $e_1$, $e_2$, and $e_3$ are all preset high-pass filtering coefficients;

extracting a maximum value from the second process sequence $(T_1, T_2...T_j...T_N)$ to generate a maximum reference value ( max ) , and extracting a minimum value from the second process sequence $(T_1, T_2...T_j...T_N)$ to generate a minimum reference value ( min ) ;

performing normalized filtering on the second process sequence $(T_1, T_2...T_j...T_N)$ to generate a third process

sequence ($P_1$, $P_2$...$P_j$...$P_N$) by setting $P_j$ according to a formula $P_j = \dfrac{T_j - min}{max - min}$, wherein the third process sequence ($P_1$, $P_2$...$P_j$...$P_N$) comprises N third process data Pj; and
setting the standard PPG data sequence as the third process sequence ($P_1$, $P_2$...$P_j$...$P_N$).

3. The blood pressure prediction method using multiple data sources according to Claim 1, wherein performing one-dimensional red light signal extraction on all the third-class PPG video frame images in the third-class PPG video frame image sequence according to a preset red light pixel threshold range to generate a first red light digital signal, and performing one-dimensional green light signal extraction on all the third-class PPG video frame images in the third-class PPG video frame image sequence according to a preset green light pixel threshold range to generate a first green light digital signal, comprise:

> Step 51, initializing the first red light digital signal to be null, initializing the first green light digital signal to be null, initializing a first index to 1, and initializing a first total number to a total number of the third-class PPG video frame images in the third-class PPG video frame image sequence;
> Step 52, setting a first-index frame image as the third-class PPG video frame image, corresponding to the first index, in the third-class PPG video frame image sequence;
> Step 53, collecting all pixels, meeting the red light pixel threshold range, in the first-index frame image to generate a red pixel set, calculating a total number of the pixels in the red pixel set to generate a total number of red pixel, calculating the sum of pixel values of all the pixels in the red pixel set to generate a red pixel value sum, and generating first-index frame red light channel data according to a quotient obtained by dividing the red pixel value sum by the red pixel total number; and adding signal points into the first red light digital signal using the first-index frame red light channel data as signal point data;
> Step 54, collecting all pixels, meeting the green light pixel threshold range, in the first-index frame image to generate a green pixel set, calculating a total number of the pixels in the green pixel set to generate a green pixel total number, calculating the sum of pixel values of all the pixels in the green pixel set to generate a green pixel value sum, and generating first-index frame green light channel data according to a quotient obtained by dividing the green pixel value sum by the green pixel total number; and adding signal points into the first green light digital signal using the first-index frame green light channel data as signal point data;
> Step 55, increasing the first index by 1;
> Step 56, determining whether the first index is greater than the first total number; if the first index is less than or equal to the first total number, performing Step 52; or, if the first index is greater than the first total number, performing Step 57; and
> Step 57, transferring the first red light digital signal to an upper processing process as a one-dimensional red light signal extraction result, and transferring the first green light digital signal to an upper processing process as a one-dimensional green light signal extraction result.

4. The blood pressure prediction method using multiple data sources according to Claim1, wherein performing maximum frequency difference determination on the second red light digital signal and the second green light digital signal to generate a first determination result, comprises:

> performing digital signal time domain-frequency domain conversion on the second red light digital signal through discrete Fourier transform to generate a red light frequency domain signal, and performing digital signal time domain-frequency domain conversion on the second green light digital signal through discrete Fourier transform to generate a green light frequency domain signal;
> extracting a maximum-energy frequency from the red light frequency domain signal to generate a maximum red light frequency, and extracting a maximum-energy frequency from the green light frequency domain signal to generate a maximum green light frequency;
> calculating a frequency difference between the maximum red light frequency and the maximum green light frequency to generate a maximum red-green frequency difference; and
> when the maximum red-green frequency difference does not exceed a preset maximum frequency difference threshold range, setting the first determination result as the up-to-standard signal identifier.

5. The blood pressure prediction method using multiple data sources according to Claim1, wherein when the first determination result is an up-to-standard signal identifier, performing signal-to-noise ratio determination on the second red light digital signal and the second green light digital signal to generate a second determination result, comprises:

when the first determination result is the up-to-standard signal identifier, according to a preset band-stop filtering frequency threshold range, removing valid signal points, meeting the band-stop filtering frequency threshold range, from the second red light digital signal through multi-order Butterworth band-stop filtering to generate a red light noise signal, and removing valid signal points, meeting the band-stop filtering frequency threshold range, from the second green light digital signal through multi-order Butterworth band-stop filtering to generate a green light noise signal;

calculating signal energy of the second red light digital signal to generate red light signal energy, calculating signal energy of the red light noise signal to generate red light noise energy, generating valid red light signal energy according to a difference between the red light signal energy and the red light noise energy, and generating a red light signal-to-noise ratio according to a ratio of the valid red light signal energy to the red light noise energy;

calculating signal energy of the second green light digital signal to generate green light signal energy, calculating signal energy of the green light noise signal to generate green light noise energy, generating valid green light signal energy according to a difference between the green light signal energy and the green light noise energy, and generating a green light signal-to-noise ratio according to a ratio of the valid green light signal energy to the green light noise energy; and

when any one of the red light signal-to-noise ratio and the green light signal-to-noise ratio is greater than or equal to the signal-to-noise threshold, setting the second determination result as the up-to-standard signal identifier.

6. The blood pressure prediction method using multiple data sources according to Claim1, wherein when the second determination result is the up-to-standard signal identifier, performing normalized PPG signal data sequence generation on the second red light digital signal and the second green light digital signal to generate the standard PPG data sequence, comprises:

when the second determination result is the up-to-standard signal identifier, performing signal data normalization processing on the second red light digital signal and the second green light digital signal respectively to generate a normalized red light signal and a normalized green light signal; setting a red light data sequence of the standard PPG data sequence as the normalized red light signal, and setting a green data sequence of the standard PPG data sequence as the normalized green light signal, wherein the standard PPG data sequence comprises the red light data sequence and the green light data sequence.

7. The blood pressure prediction method using multiple data sources according to Claim 1, wherein:

The first-class CNN model comprises multiple CNN network layers and a fully connected layer, and each CNN network layer comprises a convolutional layer and a pooling layer;

the second-class CNN model comprises a two-dimensional convolutional layer, a maximum pooling layer, a batch normalization layer, an activation layer, an add layer, a global average pooling layer, a dropout layer and a fully connected layer.

8. The blood pressure prediction method using multiple data sources according to Claim 7, wherein when the CNN model identifier is the first-class CNN identifier, selecting a first-class CNN model to perform blood pressure prediction on the standard PPG data sequence, comprises:

when the CNN model identifier is the first-class CNN identifier, performing first-class CNN model input data conversion on the standard PPG data sequence according to a preset first-class CNN input width threshold to generate an input data four-dimensional tensor;

according to a preset convolutional layer number threshold, performing multilayer convolution and pooling calculation on the input data four-dimensional tensor by means of the CNN network layers of the first-class CNN model to generate a feature data four-dimensional tensor;

performing two-dimensional matrix construction of fully connected layer input data according to the feature data four-dimensional tensor to generate an input data two-dimensional matrix, and performing feature data regression calculation on the input data two-dimensional matrix by means of the fully connected layer of the first-class CNN model to generate a blood pressure regression data two-dimensional matrix;

acquiring a preset prediction mode identifier, wherein the prediction mode identifier is a mean prediction identifier or a dynamic prediction identifier;

when the prediction mode identifier is the mean prediction identifier, performing mean blood pressure calculation on the two-dimensional matrix of blood pressure regression data to generate a mean blood pressure prediction data pair, wherein the mean blood pressure prediction data pair comprises mean systolic pressure prediction data and mean diastolic pressure prediction data; or

when the prediction mode identifier is the dynamic prediction identifier, performing dynamic blood pressure data extraction on the two-dimensional matrix of blood pressure regression data to generate a one-dimensional data sequence of dynamic blood pressure prediction .

9. The blood pressure prediction method using multiple data sources according to Claim 1, wherein when the CNN model identifier is the second-class CNN identifier, selecting a second-class CNN model to perform wavelet transform-based blood pressure prediction on the standard PPG data sequence, comprises:

when the data source identifier is the first-class PPG original signal identifier or the second-class PPG original signal identifier and the CNN model identifier is the second-class CNN identifier, performing data fragment division on the standard PPG data sequence to generate standard PPG data fragments;

acquiring a preset wavelet basis type, a scalability factor array and a mobile factor array, wherein the scalability factor array comprises H scalability factors, the mobile factor array comprises L mobile factors, and H and L are both integers;

performing signal decomposition on the standard PPG data fragments through continuous wavelet transform according to the scalability factors in the scalability factor array, the mobile factors in the scalability factor array and the wavelet basis type to generate a PPG wavelet coefficient matrix [H, L];

transforming the PPG wavelet coefficient matrix into a real matrix through a modulo operation on matrix elements, and performing normalization processing on values of matrix elements in the real matrix to generate a PPG normalized matrix [H, L];

acquiring an RGB color palette matrix, and performing PPG time-frequency tensor conversion on the PPG normalized matrix [H, L] according to the RGB color palette matrix to generate a PPG time-frequency three-dimensional tensor [H, L, 3];

according to a preset second-class CNN input width threshold, performing tensor shape reconstruction on the PPG time-frequency three-dimensional tensor [H, L, 3] through a bicubic interpolation algorithm to generate a PPG convolutional three-dimensional tensor [K, K, 3], wherein K is the second-class CNN input width threshold; and

performing blood pressure prediction on the PPG convolutional three-dimensional tensor [K, K, 3] using the second-class CNN model to generate a PPG blood pressure prediction data pair, wherein the PPG blood pressure prediction data pair comprises PPG systolic pressure prediction data and PPG diastolic pressure prediction data.

10. An equipment, comprising a memory and a processor, wherein the memory is used to store a program, and the processor is used to implement the method according to any one of Claims 1-9.

11. A computer program product comprising instructions, enabling a computer to implement the method according to any one of Claims 1-9 when running on the computer.

12. A computer-readable storage medium comprising instructions which, when executed by a computer, cause the computer to implement the method according to any one of claims 1-9.

**Patentansprüche**

1. Verfahren zur Blutdruckvorhersage unter Verwendung mehrerer Datenquellen, umfassend:
Erfassen eines Datenquellenidentifikators und Erfassen von Originaldaten von einem übergeordneten Computer (1, 101), wobei der Datenquellenidentifikator entweder ein Identifikator eines PPG-Originalsignals erster Klasse, ein Identifikator eines PPG-Originalsignals zweiter Klasse oder ein Identifikator eines PPG-Videosignals dritter Klasse ist; und die Originaldaten entweder PPG-Originaldaten erster Klasse, ein PPG-Originalsignal zweiter Klasse oder PPG-Videodaten dritter Klasse sind und dem Datenquellenidentifikator entsprechen; umfassend:

das PPG-Originalsignal erster Klasse und das PPG-Originalsignal zweiter Klasse werden über ein PPG-Signalerfassungsgerät von der Hautoberfläche einer Testperson erfasst, wobei Signale mit einer nicht offensichtlichen horizontalen Basislinienverschiebung als PPG-Originalsignale erster Klasse klassifiziert werden und Signale mit einer offensichtlichen horizontalen Basislinienverschiebung als PPG-Originalsignale zweiter Klasse klassifiziert werden, wobei die PPG-Videodaten dritter Klasse durch Fotografieren der Hautoberfläche der Testperson mit einem Videoaufzeichnungsgerät erhalten werden;
wobei der Datenquellenidentifikator so eingestellt wird, dass er den Typ der erfassten Originaldaten unterscheidet, wobei der PPG-Identifikator erster Klasse einem PPG-Originalsignal erster Klasse entspricht, der PPG-Identifikator zweiter Klasse einem PPG-Originalsignal zweiter Klasse entspricht und der PPG-Identifikator dritter Klasse PPG-Videodaten dritter Klasse entspricht;

Vorverarbeiten der Originaldaten gemäß dem Datenquellenidentifikator (2); wenn der Datenquellenidentifikator der Identifikator eines PPG-Originalsignals erster Klasse ist, Durchführen einer normalisierten Filterung des PPG-Originalsignals erster Klasse, um eine Standard-PPG-Datensequenz zu erzeugen; wenn der Datenquellenidentifikator der Identifikator eines PPG-Originalsignals zweiter Klasse ist, Entfernen der Basislinienverschiebung und Durchführen einer normalisierten Filterung des PPG-Originalsignals zweiter Klasse, um die Standard-PPG-Datensequenz zu erzeugen; und

wenn der Datenquellenidentifikator der Identifikator eines PPG-Videosignals dritter Klasse ist, Durchführen einer Videoqualitätserkennung und einer normalisierten Signalumwandlung der PPG-Videodaten dritter Klasse, um die Standard-PPG-Datensequenz zu erzeugen;

Erfassen eines CNN-Modell-Identifikators (3), wobei der CNN-Modell-Identifikator einen CNN-Identifikator erster Klasse oder einen CNN-Identifikator zweiter Klasse umfasst; und Auswählen eines entsprechenden CNN-Modells, um eine Blutdruckvorhersage für die Standard-PPG-Datensequenz gemäß des CNN-Modell-Identifikators (4) durchzuführen;

wenn der CNN-Modell-Identifikator der CNN-Identifikator erster Klasse ist, Auswählen eines CNN-Modells erster Klasse, um eine Blutdruckvorhersage für die Standard-PPG-Datensequenz durchzuführen; oder wenn der CNN-Modell-Identifikator der CNN-Identifikator zweiter Klasse ist, Auswählen eines CNN-Modells zweiter Klasse, um eine auf der Wavelet-Transformation basierende Blutdruckvorhersage für die Standard-PPG-Datensequenz durchzuführen;

**dadurch gekennzeichnet, dass**, wenn der Datenquellenidentifikator der PPG-Identifikator dritter Klasse ist, die Durchführung einer Videoqualitätserkennung und einer normalisierten Signalumwandlung an den PPG-Videodaten dritter Klasse, um die Standard-PPG-Datensequenz zu erzeugen, folgendes umfasst:

wenn der Datenquellenidentifikator der PPG-Identifikator dritter Klasse ist, die Durchführung einer Videodaten-Frame-Bild-Extraktion an den PPG-Videodaten dritter Klasse, um eine PPG-Video-Frame-Bild-Sequenz dritter Klasse zu erzeugen (102), wobei die PPG-Video-Frame-Bild-Sequenz dritter Klasse mehrere PPG-Video-Frame-Bilder dritter Klasse umfasst;

Durchführen einer eindimensionalen Rotlicht-Signalextraktion an allen PPG-Video-Frame-Bildern dritter Klasse in der PPG-Video-Frame-Bild-Sequenz dritter Klasse gemäß einem voreingestellten Rotlicht-Pixel-Schwellenbereich, um ein erstes Rotlicht-Digitalsignal zu erzeugen, und Durchführen einer eindimensionalen Grünlicht-Signalextraktion an allen PPG-Video-Frame-Bildern dritter Klasse in der PPG-Video-Frame-Bild-Sequenz dritter Klasse gemäß einem voreingestellten Grünlicht-Pixel-Schwellenbereich, um ein erstes Grünlicht-Digitalsignal zu erzeugen (103);

gemäß einem voreingestellten Bandpassfilter-Frequenzschwellenbereich Durchführen einer Bandpassfilter-Vorverarbeitung an dem ersten Rotlicht-Digitalsignal, um ein zweites Rotlicht-Digitalsignal zu erzeugen, und Durchführen einer Bandpassfilter-Vorverarbeitung an dem ersten Grünlicht-Digitalsignal, um ein zweites Grünlicht-Digitalsignal zu erzeugen (104);

Durchführen einer Bestimmung der maximalen Frequenzdifferenz für das zweite Rotlicht-Digitalsignal und das zweite Grünlicht-Digitalsignal, um ein erstes Bestimmungsergebnis zu erzeugen (105);

wenn das erste Bestimmungsergebnis ein normgerechtes Signalidentifikationszeichen ist, Durchführung einer Bestimmung des Signal-Rausch-Verhältnisses für das zweite Rotlicht-Digitalsignal und das zweite Grünlicht-Digitalsignal, um ein zweites Bestimmungsergebnis zu erzeugen; und

wenn das zweite Bestimmungsergebnis das normgerechte Signalidentifikationszeichen ist, Erzeugen einer normalisierten PPG-Signaldatensequenz aus dem zweiten Rotlicht-Digitalsignal und dem zweiten Grünlicht-Digitalsignal, um eine Standard-PPG-Datensequenz zu erzeugen,

wobei, wenn der Datenquellenidentifikator der Identifikator des PPG-Originalsignals erster Klasse ist, das Durchführen einer normalisierten Filterung des PPG-Originalsignals erster Klasse, um die Standard-PPG-Datensequenz zu erzeugen, umfasst:

wenn der Datenquellenidentifikator der Identifikator des PPG-Originalsignals erster Klasse ist, Durchführen einer Datenabtastung des PPG-Originalsignals erster Klasse gemäß einem voreingestellten Signalabtastschwellenwert erster Klasse, um eine PPG-Abtastdatensequenz erster Klasse ($X_1$, $X_2$ ...$X_i$ ...$X_M$) zu erzeugen, wobei die PPG-Abtastdatensequenz erster Klasse ($X_1$, $X_2$ ...$X_i$ ...$X_M$) M PPG-Abtastdaten erster Klasse $X_i$ umfasst, M eine ganze Zahl ist und i im Bereich von 1 bis M liegt; Durchführung einer normalisierten Filterung der PPG-Abtastdatensequenz erster Klasse ($X_1$, $X_2$ ...$X_i$ ...$X_M$), um eine erste Prozesssequenz ($Y_1$, $Y_2$ ...$Y_i$ ... $Y_M$) zu erzeugen; wenn i gleich 1 ist, wird $Y_i = X_i$ gesetzt; wenn i größer als 1 ist, wird $Y_i$ gemäß einer Formel

$$Y_i = \frac{a \times Y_{i-1}}{b} + \frac{X_i}{b \times c}$$

gesetzt, wobei die erste Prozesssequenz ($Y_1$, $Y_2$ ... $Y_i$ ... $Y_M$) M erste Prozessdaten $Y_i$ umfasst, a und b voreingestellte Filterkonstanten erster Klasse sind und c ein Verstärkungskoeffizient des ursprünglichen PPG-Signals erster Klasse ist; und

Festlegen der Standard-PPG-Datensequenz als erste Prozesssequenz ($Y_1$, $Y_2$ ... $Y_i$ ... $Y_M$).

2. Verfahren zur Blutdruckvorhersage unter Verwendung mehrerer Datenquellen gemäß Anspruch 1, wobei, wenn der Datenquellenidentifikator der Identifikator des PPG-Originalsignals zweiter Klasse ist, die Entfernung der Basislinienverschiebung und eine normalisierte Filterung des PPG-Originalsignals zweiter Klasse durchgeführt wird, um die Standard-PPG-Datensequenz zu erzeugen, umfasst:

wenn der Datenquellenidentifikator der Identifikator des PPG-Originalsignals zweiter Klasse ist, Durchführen einer Datenabtastung des PPG-Originalsignals zweiter Klasse gemäß einem voreingestellten Signalabtastschwellenwert zweiter Klasse, um eine PPG-Abtastdatensequenz zweiter Klasse ($S_1$, $S_2$ ... $S_j$ ... $S_N$) zu erzeugen, wobei die PPG-Abtastdatensequenz zweiter Klasse ($S_1$, $S_2$ ... $S_j$ ... $S_N$) N PPG-Abtastdaten zweiter Klasse $S_j$ umfasst, N eine ganze Zahl ist und j im Bereich von 1 bis N liegt;
Durchführen einer Basislinienverschiebungsentfernung und Filterung an der PPG-Abtastdatensequenz zweiter Klasse ($S_1$, $S_2$ ... $S_j$ ... $S_N$), um eine zweite Prozesssequenz ($T_1$, $T_2$ ... $T_j$ ... $T_N$) zu erzeugen; wenn j gleich 1 ist, Einstellen von

$$T_j = S_j$$

oder, wenn j größer als 1 ist, setzen von Tj gemäß einer Formel

$$T_j = e_1 \times S_j + e_2 \times S_{j-1} - e_3 \times T_{j-1}$$

wobei die zweite Prozesssequenz ($T_1$, $T_2$ ... $T_j$ ... $T_N$) N zweite Prozessdaten $T_j$ umfasst und $e_1$, $e_2$ und $e_3$ alle voreingestellte Hochpassfilterkoeffizienten sind;
Extrahieren eines Maximalwerts aus der zweiten Prozesssequenz ($T_1$, $T_2$ ... $T_j$ ... $T_N$), um einen maximalen Referenzwert (max) zu erzeugen, und Extrahieren eines Minimalwerts aus der zweiten Prozesssequenz ($T_1$, $T_2$ ... $T_j$ ... $T_N$), um einen minimalen Referenzwert (min) zu erzeugen;
Durchführen einer normalisierten Filterung der zweiten Prozesssequenz ($T_1$, $T_2$ ... $T_j$ ... $T_N$), um eine dritte

Prozesssequenz ($P_1$, $P_2$ ... $P_j$ ... $P_N$) durch Einstellen von $P_j$ gemäß einer Formel $P_j = \dfrac{T_j - min}{max - min}$ zu erzeugen, wobei die dritte Prozesssequenz ($P_1$, $P_2$ ... $P_j$ ... $P_N$) N dritte Prozessdaten Pj umfasst; und
Festlegen der Standard-PPG-Datensequenz als dritte Prozesssequenz ($P_1$, $P_2$ ... $P_j$ ... $P_N$).

3. Verfahren zur Blutdruckvorhersage unter Verwendung mehrerer Datenquellen gemäß Anspruch 1, wobei Durchführen einer eindimensionalen Rotlicht-Signalextraktion an allen PPG-Video-Frame-Bildern dritter Klasse in der PPG-Video-Frame-Bild-Sequenz dritter Klasse gemäß einem voreingestellten Rotlicht-Pixel-Schwellenbereich, um ein erstes Rotlicht-Digitalsignal zu erzeugen, und Durchführen einer eindimensionalen Grünlicht-Signalextraktion an allen PPG-Video-Frame-Bildern dritter Klasse in der PPG-Video-Frame-Bild-Sequenz dritter Klasse gemäß einem voreingestellten Grünlicht-Pixel-Schwellenbereich, um ein erstes Grünlicht-Digitalsignal zu erzeugen, umfasst:

Schritt 51: Initialisieren des ersten Rotlicht-Digitalsignals auf Null, Initialisieren des ersten Grünlicht-Digitalsignals auf Null, Initialisieren eines ersten Index auf 1 und Initialisieren einer ersten Gesamtzahl auf eine Gesamtzahl der PPG-Video-Frame-Bilder dritter Klasse in der PPG-Video-Frame-Bild-Sequenz dritter Klasse;
Schritt 52, Einstellen eines ersten Index-Bildes als PPG-Video-Frame-Bild dritter Klasse, entsprechend dem ersten Index, in der PPG-Video-Frame-Bild-Sequenz dritter Klasse;
Schritt 53: Sammeln aller Pixel, die den Schwellenbereich für Rotlicht-Pixel erfüllen, im Bild mit dem ersten Index, um einen Satz der Rotpixel zu erzeugen, Berechnen einer Gesamtzahl der Pixel im Satz Rotpixel, um eine Gesamtzahl Rotpixel zu erzeugen, Berechnen der Summe der Pixelwerte aller Pixel im Satz der Rotpixel, um eine Summe der Werte der Rotpixel zu erzeugen, und Erzeugen von Rotlichtkanaldaten des Frames mit dem ersten Index, entsprechend einem Quotienten, der durch Division der Summe der Rotpixelwerte durch die Gesamtzahl der Rotpixel erhalten wird; und Hinzufügen von Signalpunkten zum ersten Rotlicht-Digitalsignal unter Verwendung der Rotlichtkanaldaten des Frames mit dem ersten Index als Signalpunktdaten;

Schritt 54: Sammeln aller Pixel, die den Schwellenbereich für Grünlicht-Pixel erfüllen, im Bild mit dem ersten Index, um einen Satz der Grünpixel zu erzeugen, Berechnen einer Gesamtzahl der Pixel im Satz Grünpixel, um eine Gesamtzahl Grünpixel zu erzeugen, Berechnen der Summe der Pixelwerte aller Pixel im Satz der Grünpixel, um eine Summe der Werte der Grünpixel zu erzeugen, und Erzeugen von Grünlichtkanaldaten des Frames mit dem ersten Index gemäß einem Quotienten, der durch Division der Summe der Grünpixelwerte durch die Gesamtzahl der Grünpixel erhalten wird; und Hinzufügen von Signalpunkten zum ersten Grünlicht-Digitalsignal unter Verwendung der Grünlichtkanaldaten des Frames mit dem ersten Index als Signalpunktdaten;

Schritt 55: Erhöhen des ersten Index um 1;

Schritt 56: Bestimmen, ob der erste Index größer als die erste Gesamtzahl ist; wenn der erste Index kleiner oder gleich der ersten Gesamtzahl ist, Ausführen von Schritt 52; oder, wenn der erste Index größer als die erste Gesamtzahl ist, Ausführen von Schritt 57; und

Schritt 57: Übertragen des ersten Rotlicht-Digitalsignals an einen übergeordneten Verarbeitungsprozess als eindimensionales Rotlichtsignal-Extraktionsergebnis und Übertragen des ersten Grünlicht-Digitalsignals an einen übergeordneten Verarbeitungsprozess als eindimensionales Grünlichtsignal-Extraktionsergebnis.

4. Verfahren zur Blutdruckvorhersage unter Verwendung mehrerer Datenquellen gemäß Anspruch 1, wobei das Durchführen der Bestimmung der maximalen Frequenzdifferenz für das zweite Rotlicht-Digitalsignal und das zweite Grünlicht-Digitalsignal, um ein erstes Bestimmungsergebnis zu erzeugen, umfasst:

Durchführen einer Zeitdomäne-Frequenzdomäne-Umwandlung des zweiten Rotlicht-Digitalsignals durch diskrete Fourier-Transformation, um ein Rotlicht-Frequenzdomänesignal zu erzeugen, Durchführen einer Zeitdomäne-Frequenzdomäne-Umwandlung des zweiten Grünlicht-Digitalsignals durch diskrete Fourier-Transformation, um ein Grünlicht-Frequenzdomänesignal zu erzeugen; Extrahieren einer Frequenz mit maximaler Energie aus dem Rotlicht-Frequenzdomänesignal, um eine maximale Rotlichtfrequenz zu erzeugen, und Extrahieren einer Frequenz mit maximaler Energie aus dem Grünlicht-Frequenzdomänesignal, um eine maximale Grünlichtfrequenz zu erzeugen;

Berechnen einer Frequenzdifferenz zwischen der maximalen Rotlichtfrequenz und der maximalen Grünlichtfrequenz, um eine maximale Rot-Grün-Frequenzdifferenz zu erzeugen; und

wenn die maximale Rot-Grün-Frequenzdifferenz einen voreingestellten maximalen Frequenzdifferenzschwellenbereich nicht überschreitet, Setzen des ersten Bestimmungsergebnisses als normgerechtes Signalidentifikationszeichen.

5. Verfahren zur Blutdruckvorhersage unter Verwendung mehrerer Datenquellen gemäß Anspruch 1, wobei, wenn das erste Bestimmungsergebnis ein normgerechtes Signalidentifikationszeichen ist, eine Bestimmung des Signal-Rausch-Verhältnisses für das zweite Rotlicht-Digitalsignal und das zweite Grünlicht-Digitalsignal durchgeführt wird, um ein zweites Bestimmungsergebnis zu erzeugen, umfassend:

wenn das erste Bestimmungsergebnis das normgerechte Signalidentifikationszeichen ist, gemäß einem voreingestellten Bandstopp-Filterfrequenzschwellenbereich Entfernen gültiger Signalpunkte, die den Bandstopp-Filterfrequenzschwellenbereich erfüllen, aus dem zweiten Rotlicht-Digitalsignal durch mehrstufige Butterworth-Bandstoppfilterung, um ein Rotlicht-Rauschsignal zu erzeugen, und Entfernen gültiger Signalpunkte, die den Bandstopp-Filterfrequenzschwellenbereich erfüllen, aus dem zweiten Grünlicht-Digitalsignal durch mehrstufige Butterworth-Bandstoppfilterung, um ein Grünlicht-Rauschsignal zu erzeugen;

Berechnen der Signalenergie des zweiten Rotlicht-Digitalsignals, um eine Rotlichtsignalenergie zu erzeugen, Berechnen der Signalenergie des Rotlichtrauschsignals, um eine Rotlichtrauschenergie zu erzeugen, Erzeugen einer gültigen Rotlichtsignalenergie entsprechend einer Differenz zwischen der Rotlichtsignalenergie und der Rotlichtrauschenergie und Erzeugen eines Rotlicht-Signal-Rausch-Verhältnisses entsprechend einem Verhältnis der gültigen Rotlichtsignalenergie zur Rotlichtrauschenergie;

Berechnen der Signalenergie des zweiten Grünlicht-Digitalsignals, um eine Grünlichtsignalenergie zu erzeugen; Berechnen der Signalenergie des Grünlichtrauschsignals, um eine Grünlichtrauschenergie zu erzeugen, Erzeugen einer gültigen Grünlichtsignalenergie entsprechend einer Differenz zwischen der Grünlichtsignalenergie und der Grünlichtrauschenergie und Erzeugen eines Grünlicht-Signal-Rausch-Verhältnisses entsprechend einem Verhältnis der gültigen Grünlichtsignalenergie zur Grünlichtrauschenergie; und

wenn entweder das Rotlicht-Signal-Rausch-Verhältnis oder das Grünlicht-Signal-Rausch-Verhältnis größer oder gleich dem Signal-Rausch-Schwellenwert ist, Festlegen des zweiten Bestimmungsergebnisses als normgerechtes Signalidentifikationszeichen.

6. Verfahren zur Blutdruckvorhersage unter Verwendung mehrerer Datenquellen gemäß Anspruch 1, wobei, wenn das zweite Bestimmungsergebnis ein normgerechtes Signalidentifikationszeichen ist, eine normalisierte PPG-Signaldatensequenzgenerierung für das zweite Rotlicht-Digitalsignal und das zweite Grünlicht-Digitalsignal durchgeführt wird, um die Standard-PPG-Datensequenz zu erzeugen, umfassend:
wenn das zweite Bestimmungsergebnis ein normgerechtes Signalidentifikationszeichen ist, Durchführung einer Signaldaten-Normalisierungsverarbeitung für das zweite digitale Rotlicht-Signal und das zweite digitale Grünlicht-Signal, um ein normalisiertes Rotlicht-Signal und ein normalisiertes Grünlicht-Signal zu erzeugen; Festlegen einer Rotlicht-Datensequenz der Standard-PPG-Datensequenz als normalisiertes Rotlicht-Signal und Festlegen einer Grünlicht-Datensequenz der Standard-PPG-Datensequenz als normalisiertes Grün-Lichtsignal, wobei die Standard-PPG-Datensequenz die Rotlicht-Datensequenz und die Grünlicht-Datensequenz umfasst.

7. Verfahren zur Blutdruckvorhersage unter Verwendung mehrerer Datenquellen gemäß Anspruch 1, wobei:

das CNN-Modell erster Klasse mehrere CNN-Netzwerkschichten und eine vollständig verbundene Schicht umfasst und jede CNN-Netzwerkschicht eine Faltungsschicht und eine Pooling-Schicht umfasst;
das CNN-Modell zweiter Klasse eine zweidimensionale Faltungsschicht, eine Maximum-Pooling-Schicht, eine Batch-Normalisierungsschicht, eine Aktivierungsschicht, eine Additionsschicht, eine globale Durchschnitts-Pooling-Schicht, eine Dropout-Schicht und eine vollständig verbundene Schicht umfasst.

8. Verfahren zur Blutdruckvorhersage unter Verwendung mehrerer Datenquellen gemäß Anspruch 7, wobei, wenn der CNN-Modell-Identifikator der CNN-Identifikator erster Klasse ist, die Auswahl eines CNN-Modells erster Klasse zur Durchführung einer Blutdruckvorhersage für die Standard-PPG-Datensequenz umfasst:

wenn der CNN-Modell-Identifikator der CNN-Identifikator erster Klasse ist, Durchführen einer CNN-Modell-Eingabedatenkonvertierung erster Klasse für die Standard-PPG-Datensequenz gemäß einem voreingestellten CNN-Eingabebreite-Schwellenwert erster Klasse, um einen vierdimensionalen Tensor für Eingabedaten zu erzeugen;
gemäß einem voreingestellten Schwellenwert für die Anzahl der Faltungsschichten, Durchführen einer mehrschichtigen Faltung und einer Pooling-Berechnung für den vierdimensionalen Tensor der Eingabedaten mittels der CNN-Netzwerkschichten des CNN-Modells erster Klasse, um einen vierdimensionalen Tensor der Merkmalsdaten zu erzeugen;
Durchführung einer zweidimensionalen Matrixkonstruktion der vollständig verbundenen Schicht-Eingabedaten gemäß dem vierdimensionalen Tensor der Merkmalsdaten, um eine zweidimensionale Matrix der Eingabedaten zu erzeugen, und Durchführung einer Merkmalsdaten-Regressionsberechnung auf der zweidimensionalen Matrix der Eingabedaten mittels der vollständig verbundenen Schicht des CNN-Modells erster Klasse, um eine zweidimensionale Matrix der Blutdruck-Regressionsdaten zu erzeugen;
Erfassen eines voreingestellten Vorhersagemodus-Identifikators, wobei der Vorhersagemodus-Identifikator ein mittlerer Vorhersage-Identifikator oder ein dynamischer Vorhersage-Identifikator ist;
wenn der Vorhersagemodus-Identifikator der mittlere Vorhersage-Identifikator ist, Durchführung einer mittleren Blutdruckberechnung für die zweidimensionale Matrix der Blutdruckregressionsdaten, um ein mittleres Blutdruckvorhersagedatenpaar zu erzeugen, wobei das mittlere Blutdruckvorhersagedatenpaar mittlere systolische Druckvorhersagedaten und mittlere diastolische Druckvorhersagedaten umfasst; oder
wenn der Vorhersagemodus-Identifikator der dynamische Vorhersage-Identifikator ist, Durchführen einer dynamischen Blutdruckdatenextraktion aus der zweidimensionalen Matrix der Blutdruckregressionsdaten, um eine eindimensionale Datensequenz der dynamischen Blutdruckvorhersage zu erzeugen.

9. Verfahren zur Blutdruckvorhersage unter Verwendung mehrerer Datenquellen gemäß Anspruch 1, wobei, wenn der CNN-Modell-Identifikator der CNN-Identifikator zweiter Klasse ist, die Auswahl eines CNN-Modells zweiter Klasse zur Durchführung einer auf einer Wavelet-Transformation basierende Blutdruckvorhersage für die Standard-PPG-Datensequenz, umfasst:

wenn der Datenquellenidentifikator der Identifikator des PPG-Originalsignals erster Klasse oder der Identifikator des PPG-Originalsignals weiter Klasse ist und der CNN-Modell-Identifikator der CNN-Identifikator zweiter Klasse ist, Durchführung einer Datenfragmentaufteilung auf der Standard-PPG-Datensequenz, um Standard-PPG-Datenfragmente zu erzeugen;
Erfassen eines voreingestellten Wavelet-Basistyps, eines Skalierbarkeitsfaktor-Arrays und eines Mobilitätsfaktor-Arrays, wobei das Skalierbarkeitsfaktor-Array H Skalierbarkeitsfaktoren umfasst, das Mobilitätsfaktor-Array L Mobilitätsfaktoren umfasst und H und L beide ganze Zahlen sind;

Durchführen einer Signaldekomposition der Standard-PPG-Datenfragmente durch kontinuierliche Wavelet-Transformation gemäß den Skalierbarkeitsfaktoren im Skalierbarkeitsfaktor-Array, den Mobilitätsfaktoren im Skalierbarkeitsfaktor-Array und dem Wavelet-Basistyp, um eine PPG-Wavelet-Koeffizientenmatrix [H, L] zu erzeugen;

Transformieren der PPG-Wavelet-Koeffizientenmatrix in eine reelle Matrix durch eine Modulo-Operation auf Matrixelemente und Durchführen einer Normalisierungsverarbeitung an Werten von Matrixelementen in der reellen Matrix, um eine PPG-normalisierte Matrix [H, L] zu erzeugen;

Erfassen einer RGB-Farbpalettenmatrix und Durchführen einer PPG-Zeit-Frequenz-Tensor-Konvertierung an der PPG-normalisierten Matrix [H, L] gemäß der RGB-Farbpalettenmatrix, um einen PPG-Zeit-Frequenz-dreidimensionalen Tensor [H, L, 3] zu erzeugen;

gemäß einem voreingestellten Schwellenwert für die CNN-Eingabebreite zweiter Klasse, Durchführen einer Tensorformrekonstruktion für den dreidimensionalen PPG-Zeit-Frequenz-Tensor [H, L, 3] durch einen bikubischen Interpolationsalgorithmus, um einen dreidimensionalen PPG-Faltungs-Tensor [K, K, 3] zu erzeugen, wobei K der Schwellenwert für die CNN-Eingabebreite zweiter Klasse ist; und

Durchführen einer Blutdruckvorhersage für den dreidimensionalen PPG-Faltungstensor [K, K, 3] unter Verwendung des CNN-Modells zweiter Klasse, um ein PPG-Blutdruckvorhersagedatenpaar zu erzeugen, wobei das PPG-Blutdruckvorhersagedatenpaar PPG-Systoledruckvorhersagedaten und PPG-Diastoledruckvorhersagedaten umfasst.

10. Vorrichtung, die einen Speicher und einen Prozessor umfasst, wobei der Speicher zum Speichern eines Programms verwendet wird und der Prozessor zum Ausführen des Verfahrens gemäß einem der Ansprüche 1 bis 9 verwendet wird.

11. Computerprogrammprodukt, das Anweisungen umfasst, die es einem Computer ermöglichen, das Verfahren gemäß einem der Ansprüche 1 bis 9 auszuführen, wenn es auf dem Computer ausgeführt wird.

12. Computerlesbares Speichermedium, das Anweisungen umfasst, die, wenn sie von einem Computer ausgeführt werden, den Computer veranlassen, das Verfahren gemäß einem der Ansprüche 1 bis 9 auszuführen.

**Revendications**

1. Procédé de prédiction de la pression artérielle utilisant plusieurs sources de données, comprenant :

acquérir un identifiant de source de données et de données originales à partir d'un ordinateur supérieur (1, 101), dans lequel l'identifiant de source de données est l'un parmi un identifiant de signal original PPG de première classe, un identifiant de signal original PPG de deuxième classe et un identifiant vidéo PPG de troisième classe ; et les données originales sont l'une parmi des données originales PPG de première classe, un signal original PPG de deuxième classe et des données vidéo PPG de troisième classe, et correspondent à l'identifiant de source de données ; qui comprend :

le signal PPG original de première classe et le signal PPG original de deuxième classe sont acquis à partir de la surface de la peau d'un sujet testé à l'aide d'un dispositif d'acquisition de signal PPG, dans lequel les signaux présentant une dérive horizontale non évidente de la ligne de base sont classés comme des signaux PPG originaux de première classe, et les signaux présentant une dérive horizontale évidente de la ligne de base sont classés comme des signaux PPG originaux de deuxième classe, dans lequel les données vidéo PPG de troisième classe sont obtenues en photographiant la surface de la peau du sujet testé à l'aide d'un dispositif d'enregistrement vidéo ;

dans lequel l'identifiant de source de données est défini pour distinguer le type de données originales acquises, dans lequel l'identifiant PPG de première classe correspond à un signal PPG original de première classe, l'identifiant PPG de deuxième classe correspond à un signal PPG original de deuxième classe, et l'identifiant PPG de troisième classe correspond à des données vidéo PPG de troisième classe ;

prétraiter les données originales en fonction de l'identifiant de source de données (2) ;

lorsque l'identifiant de source de données est l'identifiant de signal PPG original de première classe, effectuer un filtrage normalisé sur le signal PPG original de première classe afin de générer une séquence de données PPG standard ; lorsque l'identifiant de source de données est l'identifiant de signal PPG original de deuxième classe, effectuer une suppression de dérive de base et un filtrage normalisé sur le signal PPG original de deuxième classe afin de générer la séquence de données PPG standard ; et lorsque l'identifiant de source de données est l'identifiant vidéo PPG de troisième classe, effectuer une détection de qualité vidéo et une conversion de signal normalisée sur les données vidéo PPG de troisième classe afin de générer la séquence de données PPG standard ;

acquérir un identifiant de modèle CNN (3), dans lequel l'identifiant de modèle CNN comprend un identifiant CNN de première classe ou un identifiant CNN de deuxième classe ; et

sélectionner un modèle CNN correspondant pour effectuer une prédiction de la pression artérielle sur la séquence de données PPG standard en fonction de l'identifiant de modèle CNN (4) ; lorsque l'identifiant de modèle CNN est l'identifiant CNN de première classe, sélectionner un modèle CNN de première classe pour effectuer une prédiction de la pression artérielle sur la séquence de données PPG standard ; ou lorsque l'identifiant de modèle CNN est l'identifiant CNN de deuxième classe, sélectionner un modèle CNN de deuxième classe pour effectuer une prédiction de la pression artérielle basée sur une transformée en ondelettes sur la séquence de données PPG standard ;

**caractérisé en ce que** lorsque l'identifiant de source de données est l'identifiant vidéo PPG de troisième classe, effectuer une détection de la qualité vidéo et une conversion de signal normalisée sur les données vidéo PPG de troisième classe afin de générer la séquence de données PPG standard, comprend:

lorsque l'identifiant de source de données est l'identifiant vidéo PPG de troisième classe, effectuer une extraction d'images de trames de données vidéo sur les données vidéo PPG de troisième classe afin de générer une séquence d'images de trames vidéo PPG de troisième classe (102), dans laquelle la séquence d'images de trames vidéo PPG de troisième classe comprend plusieurs images de trames vidéo PPG de troisième classe ;

effectuer une extraction de signal de lumière rouge unidimensionnel sur toutes les images vidéo PPG de troisième classe dans la séquence d'images de trames vidéo PPG de troisième classe selon une plage de seuil de pixels de lumière rouge prédéfinie afin de générer un premier signal numérique de lumiere rouge, et effectuer une extraction de signal de lumière verte unidimensionnel sur toutes les images vidéo PPG de troisième classe dans la séquence d'images de trames vidéo PPG de troisième classe selon une plage de seuil de pixels delumière verte prédéfinie afin de générer un premier signal numérique de lumière verte (103);

selon une plage de seuil de fréquence de filtrage passe-bande prédéfinie, effectuer un prétraitement de filtrage passe-bande sur le premier signal numérique de lumière rouge afin de générer un deuxième signal numérique de lumière rouge, et effectuer un prétraitement de filtrage passe-bande sur le premier signal numérique de lumière verte afin de générer un deuxième signal numérique de lumière verte (104);

effectuer une détermination de la différence de fréquence maximale sur le deuxième signal numérique de lumière rouge et le deuxième signal numérique de lumière verte afin de générer un premier résultat de détermination (105);

lorsque le premier résultat de détermination est un identifiant de signal conforme à la norme, effectuer une détermination du rapport signal/bruit sur le deuxième signal numérique de lumière rouge et le deuxième signal numérique de lumière verte afin de générer un deuxième résultat de détermination; et

lorsque le deuxième résultat de détermination est l'identifiant de signal conforme à la norme, effectuer une génération de séquence de données de signal PPG normalisée sur le deuxième signal numérique de lumière rouge et le deuxième signal numérique de lumière verte afin de générer la séquence de données PPG standard,

dans lequel, lorsque l'identifiant de source de données est l'identifiant de signal PPG original de première classe, effectuer un filtrage normalisé sur le signal PPG original de première classe afin de générer une séquence de données PPG standard, comprend:

lorsque l'identifiant de source de données est l'identifiant de signal PPG original de première classe, effectuer un échantillonnage de données sur le signal PPG original de première classe selon un seuil d'échantillonnage de signal de première classe prédéfini afin de générer une séquence de données d'échantillonnage PPG de première classe $(X_1, X_2 ... X_i ... X_M)$, dans laquelle la séquence de données d'échantillonnage PPG de première classe $(X_1, X_2 ... X_i ... X_M)$ comprend M données d'échantillonnage PPG de première classe $X_i$, M est un nombre entier et i va de 1 à M ;

effectuer un filtrage normalisé sur la séquence de données d'échantillonnage PPG de première classe $(X_1, X_2 ... X_i ... X_M)$ afin de générer une première séquence de processus $(Y_1, Y_2 ... Y_i ... Y_M)$; lorsque i est égal à 1, définir $Y_i = X_i$ ; ou, lorsque i est supérieur à 1, définir $Y_i$ selon une formule

$$Y_i = \frac{a \times Y_{i-1}}{b} + \frac{X_i}{b \times c}$$

dans lequelle la première séquence de processus $(Y_1, Y_2 ... Y_i ... Y_M)$ comprend M premières données de processus $Y_i$, a et b sont des constantes de filtrage de première classe prédéfinies, et c est un coefficient de gain du signal PPG original de première classe; et

définir la séquence de données PPG standard comme première séquence de processus $(Y_1, Y_2 ... Y_i ... Y_M)$.

2. Procédé de prédiction de la pression artérielle utilisant plusieurs sources de données selon la revendication 1, dans lequel, lorsque l'identifiant de source de données est l'identifiant de signal PPG original de deuxième classe, effectuer une suppression de dérive de base et un filtrage normalisé sur le signal PPG original de deuxième classe afin de générer la séquence de données PPG standard, comprend:

lorsque l'identifiant de la source de données est l'identifiant du signal PPG original de deuxième classe, effectuer un échantillonnage des données sur le signal PPG original de deuxième classe selon un seuil d'échantillonnage du signal de deuxième classe prédéfini afin de générer une séquence de données d'échantillonnage PPG de deuxième classe $(S_1, S_2 ... S_j ... S_N)$, dans lequelle la séquence de données d'échantillonnage PPG de deuxième classe $(S_1, S_2 ... S_j ... S_N)$ comprend N données d'échantillonnage PPG de deuxième classe Sj, N est un nombre entier et j va de 1 à N ;

effectuer une suppression de dérive de ligne de base et un filtrage sur la séquence de données d'échantillonnage PPG de deuxième classe $(S_1, S_2 ... S_j ... S_N)$ afin de générer une deuxième séquence de processus $(T_1, T_2 ... T_j ... T_N)$; lorsque j est égal à 1, définir

$$T_j = S_j$$

ou, lorsque j est supérieur à 1, définir Tj selon une formule

$$T_j = e_1 \times S_j + e_2 \times S_{j-1} - e_3 \times T_{j-1}$$

dans lequelle la deuxième séquence de processus $(T_1, T_2 ... T_j ... T_N)$ comprend N données de deuxième processus $T_j$, et $e_1$, $e_2$ et $e_3$ sont tous des coefficients de filtrage passe-haut prédéfinis;

extraire une valeur maximale de la deuxième séquence de processus $(T_1, T_2 ... T_j ... T_N)$ afin de générer une valeur de référence maximale (max), et extraire une valeur minimale de la deuxième séquence de processus $(T_1, T_2 ... T_j ... T_N)$ afin de générer une valeur de référence minimale (min);

effectuer un filtrage normalisé sur la deuxième séquence de processus $(T_1, T_2 ... T_j ... T_N)$ afin de générer une troisième séquence de processus $(P_1, P_2 ... P_j ... P_N)$ en définissant $P_j$ selon une formule $P_j = \frac{T_j - min}{max - min}$ ,

dans lequelle la troisième séquence de processus $(P_1, P_2 ... P_j ... P_N)$ comprend N troisièmes données de processus Pj; et

définir la séquence de données PPG standard comme troisième séquence de processus $(P_1, P_2 ... P_j ... P_N)$.

3. Procédé de prédiction de la pression artérielle utilisant plusieurs sources de données selon la revendication 1, dans lequel effectuer une extraction de signal de lumière rouge unidimensionnel sur toutes les images vidéo PPG de troisième classe dans la séquence d'images vidéo PPG de troisième classe selon une plage de seuil de pixels de lumière rouge prédéfinie afin de générer un premier signal numérique de lumière rouge, et l'extraction unidimensionnelle du signal de lumière verte sur toutes les images vidéo PPG de troisième classe dans la séquence d'images vidéo PPG de troisième classe selon une plage de seuil de pixels de lumière verte prédéfinie afin de générer un premier signal numérique de lumière verte, comprend :

l'étape 51, initialiser le premier signal numérique de lumière rouge à zéro, initialiser le premier signal numérique de lumière verte à zéro, initialiser un premier index à 1 et initialiser un premier nombre total au nombre total d'images de trames vidéo PPG de troisième classe dans la séquence d'images de trames vidéo PPG de troisième classe;

Étape 52, définir une image de trames d'index premier comme image de trames vidéo PPG de troisième classe, correspondant au premier index, dans la séquence d'images de trames vidéo PPG de troisième classe ;

Étape 53, collecter tous les pixels, répondant à la plage de seuil de pixels de lumière rouge, dans l'image de trame de premier index afin de générer un ensemble de pixels rouges, calculer un nombre total de pixels dans l'ensemble de pixels rouges afin de générer un nombre total de pixels rouges, calculer la somme des valeurs de pixels de tous les pixels dans l'ensemble de pixels rouges afin de générer une somme de valeurs de pixels rouges, et générer des données de trames de canal de lumière rouge de premier index selon un quotient obtenu en divisant la somme des valeurs de pixels rouges par le nombre total de pixels rouges; et ajouter des points de signal dans le premier signal numérique de lumière rouge en utilisant les données de trames de canal de lumière rouge de premier index comme données de points de signal;

Étape 54, collecter tous les pixels, répondant à la plage de seuil de pixels de lumière verte, dans l'image de la trame de premier index afin de générer un ensemble de pixels verts, calculer un nombre total de pixels dans l'ensemble de pixels verts afin de générer un nombre total de pixels verts, calculer la somme des valeurs de pixels de tous les pixels dans l'ensemble de pixels verts afin de générer une somme de valeurs de pixels verts, et générer des données de trames de canal de lumière verte de la première index selon un quotient obtenu en divisant la somme des valeurs de pixels verts par le nombre total de pixels verts; et ajouter des points de signal dans le premier signal numérique de lumière verte en utilisant les données de trames de canal de lumière verte de première index comme données de points de signal;

Étape 55, augmenter le premier index de 1;

Étape 56, déterminer si le premier index est supérieur au premier nombre total; si le premier index est inférieur ou égal au premier nombre total, exécuter l'étape 52; ou, si le premier index est supérieur au premier nombre total, exécuter l'étape 57; et

Étape 57, transférer le premier signal numérique de lumière rouge vers un processus de traitement supérieur en tant que résultat d'extraction de signal de lumière rouge unidimensionnel, et transférer le premier signal numérique de lumière verte vers un processus de traitement supérieur en tant que résultat d'extraction de signal de lumiere verte unidimensionnel.

4. Procédé de prédiction de la pression artérielle utilisant plusieurs sources de données selon la revendication 1, dans lequel effectuer la détermination de la différence de fréquence maximale sur le deuxième signal numérique de lumière rouge et le deuxième signal numérique de lumière verte afin de générer un premier résultat de détermination comprend:

effectuer une conversion du domaine temporel au domaine fréquentiel du deuxième signal numérique de lumière rouge par transformation de Fourier discrète afin de générer un signal de domaine fréquentiel de lumière rouge , et effectuer une conversion du domaine temporel au domaine fréquentiel du deuxième signal numérique de lumière verte par transformation de Fourier discrète afin de générer un signal de domaine fréquentiel de lumière verte ;

extraire une fréquence d'énergie maximale à partir du signal de domaine fréquentiel de lumière rouge afin de générer une fréquence maximale de lumière rouge, et extraire une fréquence d'énergie maximale à partir du signal de domaine fréquentiel de lumière verte afin de générer une fréquence maximale de lumière verte;

calculer une différence de fréquence entre la fréquence maximale de lumière rouge et la fréquence maximale de lumière verte afin de générer une différence de fréquence maximale entre le rouge-vert; et

lorsque la différence de fréquence maximale rouge-vert ne dépasse pas une plage de seuil de différence de fréquence maximale prédéfinie, définir le premier résultat de détermination comme identifiant de signal conforme à la norme.

5. Procédé de prédiction de la pression artérielle utilisant plusieurs sources de données selon la revendication 1, dans lequel, lorsque le premier résultat de détermination est un identifiant de signal conforme à la norme, effectuer une détermination du rapport signal/bruit sur le deuxième signal numérique de lumière rouge et le deuxième signal numérique de lumière verte afin de générer un deuxième résultat de détermination, comprenant:

lorsque le premier résultat de détermination est l'identifiant de signal conforme à la norme, conformément à une plage de seuils de fréquence de filtrage coupe-bande prédéfinie, supprimer les points de signal valides, répondant à la plage de seuils de fréquence de filtrage coupe-bande, du deuxième signal numérique de lumière rouge par le biais d'un filtrage coupe-bande Butterworth à plusieurs ordres afin de générer un signal de bruit de lumière rouge, et supprimer les points de signal valides, répondant à la plage de seuils de fréquence de filtrage coupe-bande, du deuxième signal numérique de lumière verte par le biais d'un filtrage coupe-bande Butterworth à plusieurs ordres afin de générer un signal de bruit de lumière verte;

calculer l'énergie de signal du deuxième signal numérique de lumiere rouge afin de générer l'énergie de signal rouge, calculer l'énergie du signal de bruit rouge pour générer l'énergie de bruit rouge, générer l'énergie valide du signal rouge selon la différence entre l'énergie du signal rouge et l'énergie de bruit rouge, et générer un rapport signal/bruit rouge selon le rapport entre l'énergie valide du signal rouge et l'énergie de bruit rouge;

calculer l'énergie de signal du deuxième signal numérique de lumière verte afin de générer l'énergie de signal de lumière verte, calculer l'énergie du signal de bruit de lumière verte pour générer l'énergie du bruit de lumière verte, générer une énergie de signal de lumière verte valide selon la différence entre l'énergie du signal de lumière verte et l'énergie du bruit de la lumière verte, et générer un rapport signal/bruit de la lumière verte selon le rapport entre l'énergie de signal de lumière verte valide et l'énergie du bruit de la lumière verte; et

lorsque l'un quelconque du rapport signal/bruit de lumière rouge et du rapport signal/bruit de lumière verte est supérieur ou égal au seuil signal/bruit, définir le deuxième résultat de détermination comme identifiant de signal conforme à la norme.

6.  Procédé de prédiction de la pression artérielle utilisant plusieurs sources de données selon la revendication 1, dans lequel, lorsque le deuxième résultat de détermination est l'identifiant de signal conforme à la norme, effectuer une génération de séquence de données de signal PPG normalisées sur le deuxième signal numérique de lumière rouge et le deuxième signal numérique de lumière verte afin de générer la séquence de données PPG standard, comprend: lorsque le deuxième résultat de détermination est l'identifiant de signal conforme à la norme, effectuer un processus de normalisation des données de signal sur le deuxième signal numérique de lumière rouge et le deuxième signal numérique de lumière verte respectivement afin de générer un signal de lumière rouge normalisé et un signal de lumière verte normalisé; définir une séquence de données de lumière rouge de la séquence de données PPG standard comme signal de lumière rouge normalisé, et définir une séquence de données de lumière verte de la séquence de données PPG standard comme signal de lumière verte normalisé, dans lequel la séquence de données PPG standard comprend la séquence de données de lumière rouge et la séquence de données de lumière verte.

7.  Procédé de prédiction de la pression artérielle utilisant plusieurs sources de données selon la revendication 1, dans lequel:

    le modèle CNN de première classe comprend plusieurs couches de réseau CNN et une couche entièrement connectée, et chaque couche de réseau CNN comprend une couche convolutive et une couche de regroupement;

    le modèle CNN de deuxième classe comprend une couche convolutive bidimensionnelle, une couche de regroupement maximal, une couche de normalisation par lots, une couche d'activation, une couche d'ajout, une couche de regroupement moyen global, une couche de dèfaillance et une couche entièrement connectée.

8.  Procédé de prédiction de la pression artérielle utilisant plusieurs sources de données selon la revendication 7, dans lequel, lorsque l'identifiant du modèle CNN est l'identifiant CNN de première classe, sélectionner un modèle CNN de première classe pour effectuer une prédiction de la pression artérielle sur la séquence de données PPG standard comprend:

    lorsque l'identifiant du modèle CNN est l'identifiant CNN de première classe, effectuer une conversion des données d'entrée du modèle CNN de première classe sur la séquence de données PPG standard selon un seuil de largeur d'entrée CNN de première classe prédéfini afin de générer un tenseur à quatre dimensions de données d'entrée;

    conformément à un seuil de nombre de couches convolutives prédéfini, effectuer une convolution multicouche et un calcul de regroupement sur le tenseur à quatre dimensions des données d'entrée au moyen des couches du réseau CNN du modèle CNN de première classe afin de générer un tenseur à quatre dimensions des données caractéristiques;

    effectuer une construction matricielle bidimensionnelle des données d'entrée de la couche entièrement connectée selon le tenseur de données caractéristiques à quatre dimensions afin de générer une matrice bidimensionnelle de données d'entrée, et effectuer un calcul de régression des données caractéristiques sur la matrice bidimensionnelle de données d'entrée au moyen de la couche entièrement connectée du modèle CNN de première classe afin de générer une matrice bidimensionnelle de données de régression de la pression artérielle;

    acquérir un identifiant de mode de prédiction prédéfini, dans lequel l'identifiant de mode de prédiction est un identifiant de prédiction moyenne ou un identifiant de prédiction dynamique;

    lorsque l'identifiant de mode de prédiction est l'identifiant de prédiction moyenne, effectuer un calcul de pression artérielle moyenne sur la matrice bidimensionnelle de données de régression de pression artérielle afin de générer une paire de données de prédiction de pression artérielle moyenne, dans lequelle la paire de données de prédiction de pression artérielle moyenne comprend des données de prédiction de pression systolique moyenne et des données de prédiction de pression diastolique moyenne; ou

lorsque l'identifiant de mode de prédiction est l'identifiant de prédiction dynamique, effectuer une extraction dynamique de données de pression artérielle sur la matrice bidimensionnelle de données de régression de la pression artérielle afin de générer une séquence de données unidimensionnelle de prédiction de pression artérielle dynamique.

9. Procédé de prédiction de la pression artérielle utilisant plusieurs sources de données selon la revendication 1, dans lequel, lorsque l'identifiant de modèle CNN est l'identifiant CNN de deuxième classe, sélectionner un modèle CNN de deuxième classe afin de effectuer une prédiction de la pression artérielle basée sur une transformèe en ondelettes sur la séquence de données PPG standard comprend:

lorsque l'identifiant de source de données est l'identifiant de signal PPG original de première classe ou l'identifiant de signal PPG original de deuxième classe et l'identifiant de modèle CNN est l'identifiant CNN de deuxième classe, effectuer une division de fragments de données sur la séquence de données PPG standard afin de générer des fragments de données PPG standard;

acquérir un type de base d'ondelettes prédéfini, un tableau de facteurs d'évolutivité et un tableau de facteurs mobiles, dans lequel le tableau de facteurs d'évolutivité comprend H facteurs d'évolutivité, le tableau de facteurs mobiles comprend L facteurs mobiles, et H et L sont tous deux des nombres entiers;

effectuer une décomposition du signal sur les fragments de données PPG standard par transformation en ondelettes continue selon les facteurs d'évolutivité dans le tableau de facteurs d'évolutivité, les facteurs mobiles dans le tableau de facteurs d'évolutivité et le type de base d'ondelettes afin de générer une matrice de coefficients d'ondelettes PPG [H, L];

transformer la matrice de coefficients d'ondelettes PPG en une matrice réelle par une opération modulo sur les éléments de la matrice, et effectuer un traitement de normalisation sur les valeurs des éléments de la matrice réelle afin de générer une matrice PPG normalisée [H, L];

acquérir une matrice de palette de couleurs RGB, et effectuer une conversion de tenseur temps-fréquence PPG sur la matrice PPG normalisée [H, L] selon la matrice de palette de couleurs RGB afin de générer un tenseur tridimensionnel temps-fréquence PPG [H, L, 3];

selon un seuil de largeur d'entrée CNN de deuxième classe prédéfini, effectuer une reconstruction de forme de tenseur sur le tenseur tridimensionnel temps-fréquence PPG [H, L, 3] à l'aide d'un algorithme d'interpolation bicubique afin de générer un tenseur tridimensionnel convolutif PPG [K, K, 3], dans lequel K est le seuil de largeur d'entrée CNN de deuxième classe; et

effectuer une prédiction de la pression artérielle sur le tenseur tridimensionnel convolutif PPG [K, K, 3] à l'aide du modèle CNN de deuxième classe afin de générer une paire de données de prédiction de la pression artérielle PPG, dans laquelle la paire de données de prédiction de la pression artérielle PPG comprend des données de prédiction de la pression systolique PPG et des données de prédiction de la pression diastolique PPG.

10. Équipement comprenant une mémoire et un processeur, dans lequel la mémoire est utilisée pour stocker un programme, et le processeur est utilisé pour mettre en œuvre le procédé selon l'une quelconque des revendications 1 à 9.

11. Produit logiciel comprenant des instructions permettant à un ordinateur de mettre en œuvre le procédé selon l'une quelconque des revendications 1 à 9 lorsqu'il est exécuté sur l'ordinateur.

12. Support de stockage lisible par ordinateur comprenant des instructions qui, lorsqu'elles sont exécutées par un ordinateur, amènent l'ordinateur à mettre en œuvre le procédé selon l'une quelconque des revendications 1 à 9.

Acquire a data source identifier and original data from an upper computer 1

Preprocess the original data according to the data source identifier 2

Acquire a CNN model identifier 3

Select a corresponding CNN model to perform blood pressure prediction on a standard PPG data sequence according to the CNN model identifier 4

FIG. 1

First-class PPG original signal

Second-class PPG original signal

Signal shape corresponding to a standard PPG data sequence after filtering

FIG. 2

| Acquire a data source identifier and original data from an upper computer | 101 |

↓

| When the data source identifier is a third-class PPG video identifier, perform video data frame image extraction on third-class PPG video data to generate a third-class PPG video frame image sequence | 102 |

↓

| Perform one-dimensional red light signal extraction on all third-class PPG video frame images in the third-class PPG video frame image sequence according to a preset red light pixel threshold range to generate a first red light digital signal, and perform one-dimensional green light signal extraction on all the third-class PPG video frame images in the third-class PPG video frame image sequence according to a preset green light pixel threshold range to generate a first green light digital signal | 103 |

↓

| According to a preset band-pass filtering frequency threshold range, perform band-pass filtering preprocessing on the first red light digital signal to generate a second red light digital signal, and perform band-pass filtering preprocessing on the first green light digital signal to generate a second green light digital signal | 104 |

↓

| Perform maximum frequency difference determination on the second red light digital signal and the second green light digital signal to generate a first determination result which is set as not-up-to-standard signal identifier | 105 |

↓

| If the first determination result is the not-up-to-standard signal identifier, stop the PPG signal processing process, and return warning information indicating that the quality of the PPG original signal is not up to standard to the upper computer | 106 |

FIG. 3

Memory ↔ Processor

FIG. 4

**EP 4 122 382 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010189221

**Non-patent literature cited in the description**

- **BAEK SANGHYUN et al.** End-to-End Blood Pressure Prediction via Fully Convolutional Networks. IEEE ACCESS, 31 December 2019, vol. 7